# FASCICULE DE BREVET EUROPEEN

(11) **EP 0 556 080 B1**
(45) Date de publication et mention de la délivrance du brevet: **08.05.2002**
(21) Numéro de dépôt: 93400194.2
(22) Date de dépôt: 27.01.1993
(51) Int. Cl.: C07D 471/04, A61K 31/44

(54) **Dérivés bicycliques de la pyridine, leur procédé de préparation, les intermédiaires obtenus, leur application à titre de médicaments et les compositions pharmaceutiques les renfermant**
Bicyclische Pyridinderivate, Verfahren zu ihrer Herstellung, Zwischenprodukte, ihre Anwendung als Arzneimittel und diese enthaltende pharmazeutische Zusammensetzungen
Bicyclic pyridine derivatives, process for their preparation, intermediates, their use as medicaments and pharmaceutical compositions containing them

(30) Priorité: 31.01.1992 FR 9201084
(43) Date de publication de la demande: 18.08.1993
(73) Titulaire: Aventis Pharma S.A., 92160 Antony (FR)
(72) Inventeur: Corbier, Alain, F-91370 Verrieres le Buisson (FR); Fortin, Michel, F-75018 Paris (FR); Guillaume, Jacques, F-93190 Livry Gargan (FR); Haesslein, Jean-Luc, F-77181 Courtry (FR); Vevert, Jean-Paul, F-93500 Pantin (FR)

(56) Documents cités:
- EP-A- 0 121 197
- EP-A- 0 266 890
- EP-A- 0 356 234
- EP-A- 0 404 190
- EP-A- 0 461 040
- WO-A-91/15479
- FR-A- 2 647 451
- CHEM. PHARM. BULL. vol. 34, no. 7, 1986, pages 2833 - 2839 K. AWANO ET AL
- CHEMICAL ABSTRACTS, vol. 102, 1985, Columbus, Ohio, US; abstract no. 149262u,
- CHEM. PHARM. BULL. vol. 34, no. 7, 1986, pages 2828 - 2832 K. AWANO ET AL
- CHEMICAL ABSTRACTS, vol. 86, 1977, Columbus, Ohio, US; abstract no. 5469v,
- HETEROCYCLES vol. 23, no. 6, 1985, pages 1395 - 1398 I. YOKOE ET AL
- ARZNEIM.-FORSCH. vol. 42, no. 1, 1992, pages 48 - 55 M. KOMURO ET AL
- SYNTHESIS 1984, pages 263 - 265 S. PODERGAJS ET AL
- CHEMICAL ABSTRACTS, no. 64, 1966, Columbus, Ohio, US; abstract no. 5069c,
- CHEMICAL ABSTRACTS, vol. 81, 1974, Columbus, Ohio, US; abstract no. 136105t,
- CHEMICAL ABSTRACTS, vol. 112, 1990, Columbus, Ohio, US; abstract no. 77097a,
- CHEMICAL ABSTRACTS, vol. 115, 1991, Columbus, Ohio, US; abstract no. 71478r,
- J. ORG. CHEM. vol. 41, no. 22, 1976, pages 3549 - 3556 E.S. HAND ET AL
- J. LABELLED. COMPD. RADIOPHARM. vol. 22, no. 7, 1985, pages 735 - 743 Y. NAGATSU ET AL
- SYNTHESIS 1988, pages 199 - 203 K. BURGER ET AL
- J. MED. CHEM. vol. 35, no. 5, 1992, pages 877 - 885 A.P. THOMAS ET AL

## Description

La présente invention concerne de nouveaux dérivés bicycliques de la pyridine, leur procédé de préparation, les nouveaux intermédiaires obtenus, leur application à titre de médicaments et les compositions pharmaceutiques les renfermant.
- Les articles CHEM. PHARM BULL. Vol. 34, no. 7, 1986, pages 2833-2839 K. AWANO ET AL et pages 2828-2832 décrivent des dérivés de pyrazolo [1,5-a] pyridine substitués en 3 ayant une activité inhibitrice sur l'agrégation plaquettaire.
- CHEMICAL ABSTRACTS (CA), vol. 102, 1985, no. 149262u, & JP-A-59 175 491 ainsi que EP-A-0 121 197 décrivent des dérivés de pyrazolopyridine comme inhibiteur de l'agrégation plaquettaire.
   Le brevet EP-A-0 266 890 décrit des dérivés
   d'imidazopyridine utiles notamment pour traiter les désordres gastriques.
- CA, vol. 86, 1977, no. 5469v, & SU-A-522 187 décrivent des dérivés 3-acyl-2-alkyl (aryl) imidazo [1,2-a] pyridine et leur préparation.
- L'article HETEROCYCLES vol. 23, no. 6, 1985, pages 1395-1398 I. YOKOE ET AL décrit des dérivés de pyrazolopyridine préparés par cycloaddition de pyridinium N-ylides avec chromones et coumarin.
- L'article ARZNEIM.-FORSCH. Vol. 42, no. 1, 1992, pages 48-55 M. KOMURO ET AL décrit des dérivés 2 méthyl-3-(1,4,5,6-tetrahydronicotinoyl) pyrazolo [1,5-a] pyridine comme métabolites urinaires chez le rat, le lapin et le chien.
- L'article SYNTHESIS, 1984, pages 263-265 S.PODERGAJS ET AL décrit notamment la préparation d'imidazo [1,2-a] pyrimidine.
- Le brevet FR-A- 2647 451 décrit des dérivés d'imidazo [1,2-a] pyridine utiles notamment pour leurs propriétés bradycardisantes, hypotensives et anesthésiques locales.
- Le brevet EP-A-0 356 234 décrit des dérivés de benzazole ayant notamment une activité anti-ulcéreuse.
- CA, no. 64, 1966, no. 5069c & J. ORG CHEM., Vol. 30, no.12, 1965, pages 4085-4090 J.P. PAOLINI ET AL décrivent des dérivés de l'imidazo [1,2-a] pyridine et leur préparation.
- CA, vol. 81, 1974, no. 136105t & YAKUGAKU ZASSHI vol. 94, no. 8, 1974, pages 952-963 K. KUSUGA ET AL décrivent des réactions de dérivés de N-aminopyridinium et de pyrazolodiazines pour préparer notamment des dérivés de pyrazolo pyridines.
- CA, vol. 112, 1990, no. 77097a & DOKL. AKAD. NAUK UKR. SSR, SER. B: GEOL., KHIM. BIOL. NAUKI no. 5, 1989, pages 36-38 G.P.KUTROV ET AL décrivent des réactions notamment de 2-amino pyridine et 2-aminopyrinidines avec 1-3-dibromoacétone pour donner notamment des dérivés de bromure d'imidazolium.
- CA, vol. 115, 1991, no. 71478r, & UKR. KHIM. ZH. (RUSS. ED.) Vol. 57, no. 2, 1991, pages 187-191 G.P. KUTROV ET AL décrivent synthèse et propriétés de dérivés notamment vinylimidazo (1,2-a) pyridines et (1,2-a) pyrimidines.
- L'article J. ORG. CHEM. Vol.41, no.22, 1976, pages 3549-3556 E.S. HAND ET AL décrit des réactions de substitution sur des dérivés imidazo (1,2-a) pyridines.
- L'article J. LABELLED. COMPD. RADIOPHARM. Vol.22, no. 7, 1985, pages 735-743 Y. NAGATSU ET AL décrit la synthèse et le marquage de dérivés pyrazolo (1,5-a) pyridine.
- L'article SYNTHESIS 1988, pages 199-203 K. BURGER ET AL décrit des réactions chimiques pour préparer notamment des imidazo (1,2-a) pyridines.
- Le brevet EP-A-0 404 190 décrit la synthèse d'hétérocycles condensés notamment de dérivés imidazo (1,2-a) pyridine.
- Le brevet EP 0461040 décrit des dérivés de l'imidazole ayant des propriétés antagonistes pour le récepteur de l'Angiotensine II.

La présente invention a pour objet :
a) les produits de formule (I) : 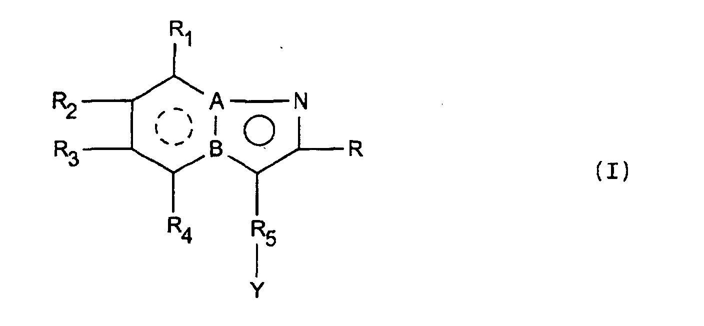 dans laquelle :
   l'un de A ou B représente un atome d'azote et l'autre représente un atome de carbone tel que l'hétérobicycle ainsi formé représente un radical imidazo pyridine, pyrazolo pyridine ou pyrazolo tétrahydro imidazotétrahydropyridine, pyridine,
   R représente un radical n-butyle ou méthyle,
   R₁, R₂, R₃ et R₄ sont tels que trois d'entre eux représentent un atome d'hydrogène et l'autre représente un atome d'hydrogène, un radical carboxy libre ou estérifié par un radical alkyle linéaire ou ramifié renfermant au plus 4 atomes de carbone,
   R₅ représente un radical méthylène ou un radical -C=O,
   et Y représente le radical phényle ou biphényle substitué par un atome d'halogène, un radical cyano, carboxy libre ou estérifié par un radical alkyle ou un radical tétrazolyle, les radicaux alkyle étant linéaires ou ramifiés et renfermant au plus 4 atomes de carbone, à l'exception des produits dans lesquels R₁, R₂, R₃ et R₄ représentent un atome d'hydrogène
   A représente N,
   R représente un radical alkyle,
   R₅ représente -CO
   et Y représente le radical phényle substitué par un atome d'halogène.
b) Les produits suivants :
   - (4-bromophényl) (2-butyl pyrazolo (1,5-a) pyridin-3-yl) méthanone
   - (4-bromophényl) (2-butyl 7- méthyl pyrazolo (1,5-a) pyridin-3-yl) méthanone lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).
      Dans les produits de formule (I) et dans ce qui suit, le bicycle formé par : 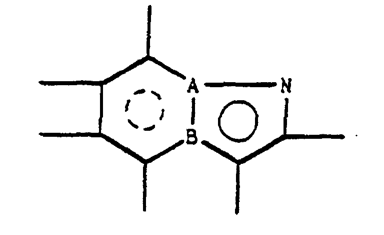
dans lequel A, B et les traits pointillés et continus ont la signification indiquée ci-dessus, les doubles liaisons le cas échéant, se situant différemment selon que A ou B représente un atome d'azote, peut ainsi représenter les bicycles suivants : 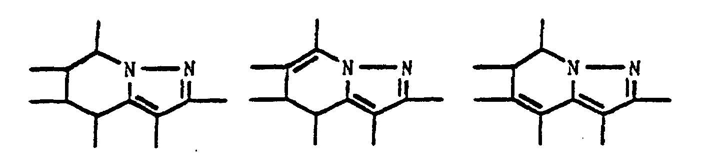 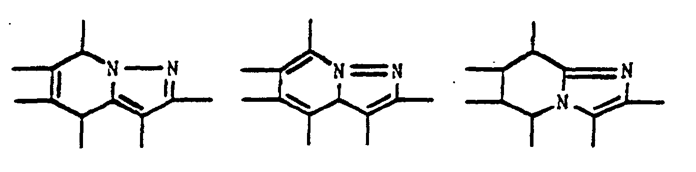 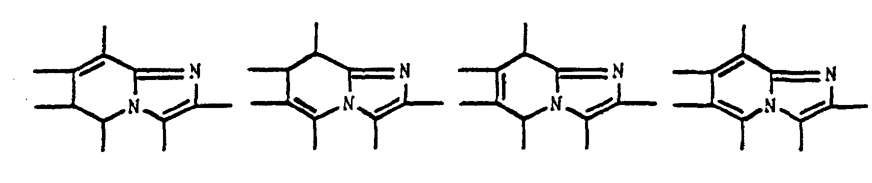 les bicycles représentés ci-dessus portant les substituants R, R₁, R₂, R₃, R₄, R₅ et Y dont les significations et l'emplacement sur ces bicycles sont ceux indiqués ci-dessus dans les produits de formule (I).

Dans les produits de formule (I) et dans ce qui suit :
- le terme radical alkyle linéaire ou ramifié désigne de préférence les radicaux méthyle, éthyle, propyle, isopropyle, butyle, isobutyle, sec-butyle et tert-butyle mais peut également représenter un radical pentyle ou hexyle et particulièrement isopentyle et isohexyle,
- le terme radical alkényle linéaire ou ramifié désigne de préférence un radical vinyle, allyle, 1-propényle, butényle et particulièrement 1-butényle, ou pentényle,
- le terme radical alkynyle linéaire ou ramifié désigne de préférence un radical éthynyle, propargyle, butynyle ou pentynyle,
- le terme atome d'halogène désigne de préférence l'atome de chlore, mais peut aussi représenter un atome de fluor, de brome ou d'iode,
- le terme radical alcoxy linéaire ou ramifié désigne de préférence les radicaux méthoxy, éthoxy, propoxy ou isopropoxy, mais peut aussi représenter un radical butoxy linéaire, secondaire ou tertiaire,
- le terme radical acyle désigne de préférence un radical ayant de 1 à 6 atomes de carbone tel que par exemple le radical formyle, acétyle, propionyle, butyryle ou benzoyle, mais également le radical pentanoyle, hexanoyle, acryloyle, crotonoyle ou carbamoyle,

Dans le terme amino substitué par un ou deux radicaux alkyle ou alkényle, ces radicaux sont choisis parmi les radicaux alkyle et alkényle tels que définis ci-dessus tels que par exemple méthyl ou éthylamino, ou encore diméthylamino ou méthyléthylamino,
- le terme radical acyloxy désigne par exemple un radical dans lequel le radical acyle a les valeurs indiquées ci-dessus et désigne de préférence un radical formyloxy, acétyloxy, propionyloxy, butyryloxy ou benzoyloxy,
- le terme radical cycloalkyle désigne de préférence les radicaux cyclopropyle, cyclobutyle, cyclopentyle ou cyclohexyle.

Dans les produits de formule (I) et dans ce qui suit :
- comme exemples de radical alkyle tel que défini ci-dessus et substitué par un ou plusieurs atomes d'halogène tel que défini ci-dessus, on peut citer par exemple les radicaux bromoéthylthio, trifluorométhylthio, trifluoroéthylthio ou encore pentafluoroéthylthio,
- comme exemples de radical alcoxy tel que défini ci-dessus et substitué par un ou plusieurs atomes d'halogène tel que défini ci-dessus on peut citer, par exemple les radicaux bromoéthoxy, trifluorométhoxy, trifluoroéthoxy ou encore pentafluoroéthoxy,
- le terme radical carbamoyle désigne également les radicaux carbamoyle substitué par exemple un groupe N-monoalkyl inférieur carbamoyle, tel que N-méthylcarbamoyle, N-éthylcarbamoyle, un groupe N,N-dialkyl inférieur carbamoyle, tel que N,N-diméthylcarbamoyle, N,N-diéthylcarbamoyle ; un groupe N-(hydroxyalkyl inférieur) carbamoyle, tel que N-(hydroxyméthyl) carbamoyle, N-(hydroxyéthyl) carbamoyle, un groupe carbamoylalkyle inférieur, tel que carbamoylméthyle, carbamoyléthyle,
- le terme radical acyloxy désigne les radicaux dans lesquels les radicaux acyle ont la signification indiquée ci-dessus et par exemple de préférence les radicaux acétoxy ou propionyloxy,

Le ou les radicaux carboxy des produits de formule (I) peuvent être salifiés, estérifiés ou amidifiés par les groupements divers connus de l'homme de métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle,
- parmi les composés d'amidification, les radicaux
- CO₂-NH-COOH, -CO₂-NH-COOaryle, -CO₂-NH-COOalkyle,
- CO₂-NH-SO₂-Oalkyle, -CO₂-NH-SO₂-Oaryle, -CO₂-NH-SO₂-N(alkyle)₂, dans lesquels les radicaux alkyle et aryle ont les significations indiquées ci-dessus pour ces radicaux et sont éventuellement substitués ainsi qu'il est également indiqué ci-dessus et notamment aryle représente phényle et tétrazolyle éventuellement salifié.

Par radical carboxy amidifié, on entend également le radical : 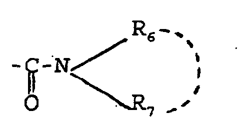 dans lequel R₆ et R₇ ont la signification précédente.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoique, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Parmi les produits objet de l'invention, peuvent être cités tout particulièrement les produits de formule (I) répondant aux formules suivantes :
- l'acide 2-butyl 3-[(2'-1H-tétrazol 5-yl) (1,1'-biphényl) 4-yl) méthyl] pyrazolo-(1,5-a)pyridine 4-carboxylique,
- l'acide 4'-[(2-butyl pyrazolo-(1,5-a)pyridin 3-yl) méthyl] (1,1'-biphényl) 2-carboxylique,
- l'acide 4'-[(2-butyl imidazo-(1,2-a)pyridin 3-yl) méthyl] (1,1'-biphényl) 2-carboxylique,
- l'acide 4-[(2-butyl imidazo-(1,2-a)pyridin 3-yl) méthyl] benzoïque,
   ainsi que les sels d'addition avec les acides minéraux et organiques ou avec les bases minérales et organiques.

L'invention a également pour objet un procédé de préparation de produits de formule (I)ₘ qui correspondent aux produits de formule (I) telle que définie ci-dessus, dans laquelle R₅ représente un radical 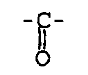 ou méthylène, caractérisé en ce que l'on fait réagir un composé de formule (II) : 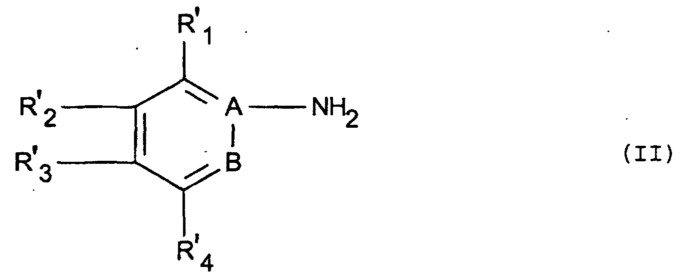 dans laquelle A et B ont la signification indiquée ci-dessus et R'₁, R'₂, R'₃ et R'₄ ont les significations indiquées ci-dessus respectivement pour R₁, R₂, R₃ et R₄ dans lesquelles les éventuelles fonctions réactives sont éventuellement protégées par des groupements protecteurs, avec un composé de formule (III) : 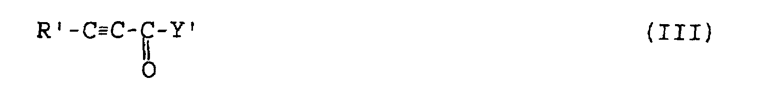 dans laquelle R' et Y' ont les significations indiquées ci-dessus pour R et Y dans lesquelles les éventuelles fonctions réactives sont éventuellement protégées par des groupements protecteurs, pour obtenir un produit de formule (IV) : 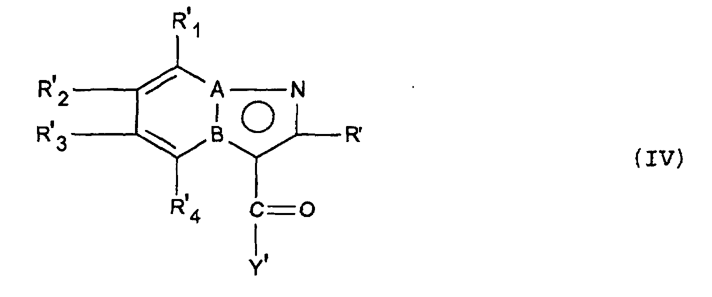 dans laquelle A, B, R', R'₁, R'₂, R'₃, R'₄ et Y' ont les significations indiquées ci-dessus, que l'on soumet éventuellement à une réaction de réduction pour obtenir un produit de formule (V) : 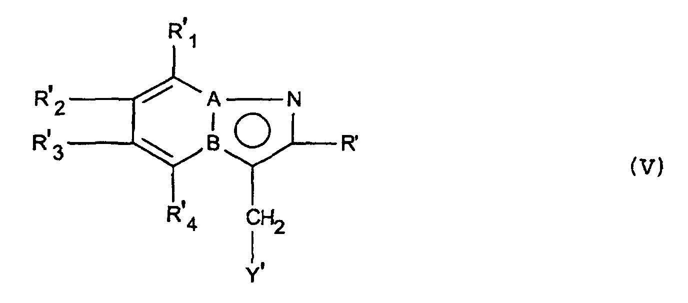 dans laquelle R', R'₁, R'₂, R'₃, R'₄ et Y' ont les significations indiquées ci-dessus, produits de formules (IV) et (V) que l'on traite, si désiré et si nécessaire, à l'une ou plusieurs des réactions suivantes, dans un ordre quelconque :
- une réaction d'élimination des groupements protecteurs que peuvent porter les fonctions réactives protégées,
- une réaction de salification par un acide minéral ou organique ou par une base pour obtenir le sel correspondant,
- une réaction d'hydrogénation du cycle pyridinyl porteur des groupes A et B,
- une réaction de substitution d'un atome d'halogène par un radical cyano,
- une réaction de substitution d'un atome d'halogène par un radical alkyle ou aryle éventuellement substitué,
- une réaction de transformation de fonction cyano en fonction acide,
- une réaction de transformation de fonction cyano en fonction tétrazolyle,
- une réaction d'estérification ou salification de fonction acide,
- une réaction de transformation de radical formyle en radical carbamoyle,
- une réaction de transformation de radical carbamoyle en radical cyano,
- une réaction de saponification de fonction ester en fonction acide,
- une réaction de transformation de fonction alcoxy en fonction hydroxyle,
- une réaction de réduction de la fonction carboxy en fonction alcool,
- une réaction de réduction de la fonction oxo en radical alkylène,
- une réaction d'oxydation de radical alkylène en fonction oxo
- une réaction de transformation de radicaux alkylthio ou arylthio en les radicaux sulfoxyde ou sulfone correspondants,
- une réaction de dédoublement des formes racémiques en produits dédoublés,
   lesdits produits de formule (I)ₘ ainsi obtenus, qui correspondent aux produits de formule (I) telle que définie ci-dessus dans laquelle R₅ représente un radical 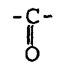
ou méthylène, étant sous toutes les formes isomères possibles racémiques, énantiomères et diastéréoisomères.

L'invention a également pour objet un procédé de préparation de produits de formule (I) telle que définie ci-dessus, caractérisé en ce que l'on fait réagir un composé de formule (II) : 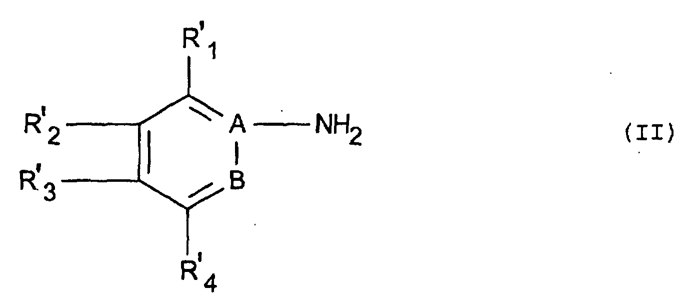 dans laquelle A et B ont la signification indiquée ci-dessus, et R'₁, R'₂, R'₃ et R'₄ ont les significations indiquées ci-dessus, respectivement pour R₁, R₂, R₃ et R₄ dans lesquelles les éventuelles fonctions réactives sont éventuellement protégées par des groupements protecteurs, avec un composé de formule (VI) : 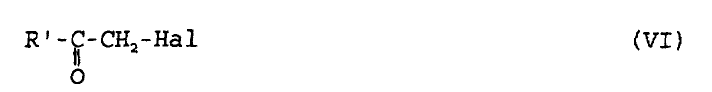 dans laquelle R' a la signification indiquée ci-dessus pour R, dans laquelle les éventuelles fonctions réactives sont éventuellement protégées par des groupements protecteurs et Hal représente un atome d'halogène, pour obtenir un produit de formule (VII) : 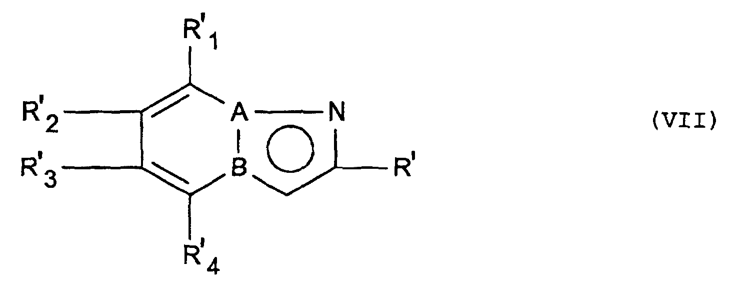 dans laquelle A, B, R', R'₁, R'₂, R'₃ et R'₄ ont les significations indiquées ci-dessus,
que l'on soumet à une réaction d'halogénation pour obtenir un produit de formule (VIII) : 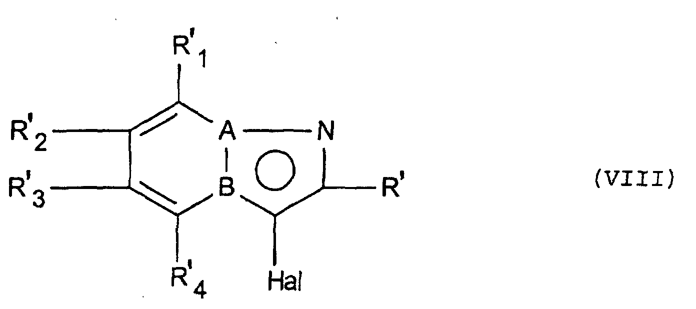 dans laquelle A, B, R', R'₁, R'₂, R'₃ et R'₄ ont les significations indiquées ci-dessus et Hal représente un atome d'halogène, que l'on fait réagir avec le composé de formule (IX) :

Br-Zn-R₅-Y' (IX)

dans laquelle Y' a la signification indiquée ci-dessus, pour Y dans laquelle les éventuelles fonctions réactives sont éventuellement protégées par des groupements protecteurs et R₅ a la signification indiquée ci-dessus, pour obtenir un produit de formule (X) : 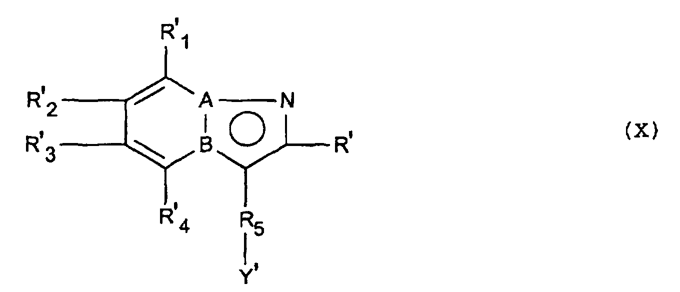 dans laquelle A, B, R', R'₁, R'₂, R'₃, R'₄, R₅ et Y' ont les significations indiquées ci-dessus, produits de formule (X) que l'on traite, si désiré et si nécessaire, à l'une ou plusieurs des réactions suivantes, dans un ordre quelconque :
- une réaction d'élimination des groupements protecteurs que peuvent porter les fonctions réactives protégées,
- une réaction de salification par un acide minéral ou organique ou par une base pour obtenir le sel correspondant,
- une réaction d'hydrogénation du cycle pyridinyle,
- une réaction de substitution d'un atome d'halogène par un radical cyano,
- une réaction de substitution d'un atome d'halogène par un radical aryle éventuellement substitué,
- une réaction de transformation de fonction cyano en fonction acide,
- une réaction de transformation de fonction cyano en fonction tétrazolyle,
- une réaction d'estérification ou salification de fonction acide,
- une réaction de transformation de radical formyle en radical carbamoyle,
- une réaction de transformation de radical carbamoyle en radical cyano,
- une réaction de saponification de fonction ester en fonction acide,
- une réaction de transformation de fonction alcoxy en fonction hydroxyle,
- une réaction de réduction de la fonction carboxy en fonction alcool,
- une réaction de réduction de la fonction oxo en radical alkylène,
- une réaction d'oxydation de radical alkylène en fonction oxo
- une réaction de transformation de radicaux alkylthio ou arylthio en les radicaux sulfoxyde ou sulfone correspondants,
- une réaction de dédoublement des formes racémiques en produits dédoublés,
lesdits produits de formule (I) ainsi obtenus étant sous toutes les formes isomères possibles racémiques, énantiomères et diastéréoisomères.

Dans des conditions préférentielles de mise en oeuvre de l'invention :
- le produit de formule (IV) peut être obtenu par addition du composé de formule (III) sur la fonction amine libre du composé de formule (II), suivie d'une réaction de cyclisation. Ce procédé s'applique à la formation de tous les produits de formule (I), mais s'applique préférentiellement à la préparation des produits de formule (I) dans lesquels A représente un atome d'azote et B un atome de carbone. La réaction d'obtention du composé de formule (IV) peut s'écrire de la façon suivante :

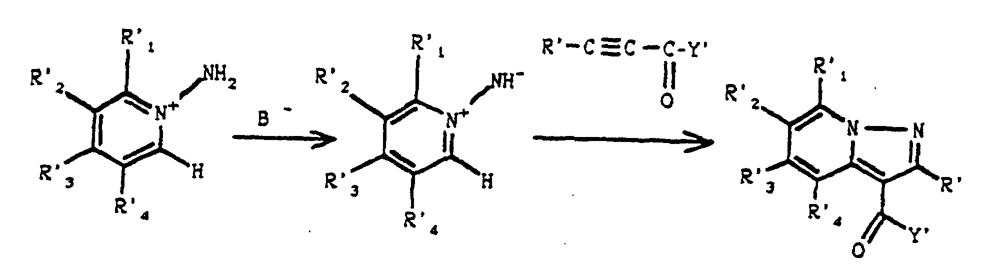

La réaction du produit de formule (III) sur le produit de formule (II) peut être réalisée dans un solvant tel que par exemple l'acétonitrile, le diméthylformamide ou encore le tétrahydrofuranne, le diméthoxyéthane ou le diméthylsulfoxyde au reflux du solvant ou à la température ambiante, de préférence sous agitation ; la réaction est réalisée en présence d'une base telle que par exemple l'hydrure de sodium ou de potassium ou encore du carbonate de sodium ou de potassium, du méthylate ou éthylate ou tert-butylate de sodium ou de potassium.

La fonction oxo des produits de formule (IV) peut être réduite en radical méthylène pour donner des produits de formule (V), dans les conditions usuelles connues de l'homme de métier, telles que par exemple en présence d'hydrogène et palladium, dans un solvant tel que par exemple de l'acide acétique, de l'acide perchlorique, ou en présence d'hydrure de lithium et d'aluminium, dans un solvant tel que l'éther éthylique de préférence en présence d'un acide de Lewis tel que le chlorure d'aluminium.

On essore au moyen de l'hydrate d'hydrazine en présence de potasse dans l'éthylène glycol à 140-210°C, selon Wolff-Kishner.

Dans le composé de formule (VI), l'atome d'halogène peut représenter par exemple, un atome de chlore ou de fluor et de préférence un atome de brome.

La réaction d'addition du composé de formule (VI) sur le composé de formule (II) pour obtenir le produit de formule (VII) peut être réalisée dans un solvant tel que l'acétone au reflux.

Cette réaction s'applique particulièrement lorsque le composé de formule (II) est tel que A représente un atome de carbone et B représente un atome d'azote.

Le produit de formule (VII) obtenu peut être soumis à une réaction d'halogénation pour donner le produit de formule (VIII).

Dans cette réaction, l'atome d'halogène peut être un atome de chlore ou de brome.

La réaction de bromation peut être réalisée par exemple en présence de N-bromo succinimide dans un solvant tel que par exemple le chloroforme.

La réaction d'addition du composé de formule (IX) sur le produit de formule (VIII) peut être réalisée dans les conditions usuelles connues de l'homme de métier, de la réaction d'un organo métallique qui peut être un magnésien ou un zincique sur un atome d'halogène dans un solvant tel que par exemple du tétrahydrofuranne.

Selon les valeurs de R', R'₁, R'₂, R'₃, R'₄ et Y', les produits de formule (IV), (V) et (X) constituent ou non des produits de formule (I).

Les diverses fonctions réactives que peuvent porter certains composés des réactions définies ci-dessus peuvent, si nécessaire, être protégées ; il s'agit par exemple des radicaux hydroxyle, acyle, carboxy libres ou encore amino et mono-alkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals cycliques ou non cycliques tels que le diméthyl ou diéthylcétal ou l'éthylène dioxycétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :

- les fonctions acide peuvent être protégées par exemple sous forme d'esters formés avec les esters facilement clivables tels que les esters méthyliques, éthyliques, benzyliques ou ter butyliques ou des esters connus dans la chimie des peptides.

Les réactions auxquelles les produits de formule (IV), (V) ou (X) telle que définie ci-dessus peuvent être soumis, si désiré ou si nécessaire, peuvent être réalisées, par exemple, comme indiqué ci-aprés :
- L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, dans un alcanole formique ou trifluoroacétique ou alcaline avec de la soude ou de la potasse ou encore par une hydrogénation catalytique.
   Le groupement phtalimido peut être éliminé par l'hydrazine.
   On trouvera une liste de différents groupements protecteurs utilisables par exemple dans le brevet BF 2 499 995.
   Les produits décrits ci-dessus peuvent, si désiré, faire l'objet de réactions de salification par exemple par un acide minéral ou organique selon les méthodes usuelles connues de l'homme de métier.
- Les produits décrits ci-dessus peuvent, si désiré, faire l'objet, sur les éventuelles fonctions carboxy, de réactions de salification par une base minérale ou organique ou d'estérification : ces réactions d'estérification et de salification peuvent être réalisées selon les méthodes usuelles connues de l'homme de métier.
- Les éventuelles transformations de fonctions ester en fonction acide des produits décrits ci-dessus peuvent être, si désiré, réalisées dans les conditions usuelles connues de l'homme de métier notamment par hydrolyse acide ou alcaline par exemple par de la soude ou de la potasse en milieu alcoolique tel que, par exemple, dans du méthanol ou encore par de l'acide chlorhydrique ou sulfurique.
- Les éventuelles fonctions cyano des produits décrits ci-dessus peuvent être, si désiré, transformées en fonction acide dans les conditions usuelles connues de l'homme de métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.
- Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme de métier et notamment par de l'hydrure de lithium et d'aluminium dans un solvant tel que par exemple le tétrahydrofuranne ou encore le dioxane ou l'éther éthylique.

Les éventuelles fonctions carboxy libre des produits décrits ci-dessus peuvent être, si désiré, réduites en fonction alcool notamment par de l'hydrure de bore.
- Les éventuelles fonctions alcoxy telles que notamment méthoxy des produits décrits ci-dessus peuvent être, si désiré, transformées en fonction hydroxyle dans les conditions usuelles connues de l'homme de métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide acétique au reflux.
- Les éventuels groupements alkylthio ou arylthio des produits décrits ci-dessus peuvent être, si désiré, transformées en les fonctions sulfoxyde ou sulfone correspondantes dans les conditions usuelles connues de l'homme de métier telles que par exemple par les peracides comme par exemple l'acide peracétique ou l'acide métachloroperbenzoïque ou encore par l'ozone, l'oxone, le périodate de sodium dans un solvant tel que par exemple le chlorure de méthylène ou le dioxanne à la température ambiante.
   L'obtention de la fonction sulfoxyde peut être favorisée par un mélange équimolaire du produit renfermant un groupement alkylthio ou arylthio et du réactif tel que notamment un peracide.
   L'obtention de la fonction sulfone peut être favorisée par un mélange du produit renfermant un groupement alkylthio ou arylthio avec un excès du réactif tel que notamment un peracide.
   La réaction de substitution d'un atome d'halogène par un radical cyano peut être effectuée dans les conditions usuelles connues de l'homme de métier et notamment par réaction de cyanure cuivreux au reflux du diméthylformamide.
   La réaction d'hydrogénation du cycle pyridinyl peut être réalisée suivant les méthodes usuelles connues de l'homme de métier et notamment par hydrogénation catalytique en présence de palladium dans un mélange d'acide acétique et d'acide perchlorique ou encore d'oxyde platine dans l'acide acétique.
   La réaction de substitution d'un atome d'halogène par un radical aryle éventuellement substitué peut être réalisée dans les conditions usuelles connues de l'homme de métier et notamment par un dérivé métallique, magnésien ou zincique ou organo stanneux sur un atome de brome par exemple ainsi qu'il est indiqué ci-après dans la partie expérimentale.
- La réaction d'oxydation de radical alkylène en radical oxo peut être réalisée dans les conditions usuelles connues de l'homme de métier.
- La réduction de la fonction oxo en radical alkylène peut être effectuée selon les méthodes usuelles connues de l'homme de métier et notamment par celles décrites pour l'obtention de produits de formule (V) à partir de produits de formule (IV).
- Les éventuelles fonctions alcool des produits décrits ci-dessus peuvent être, si désiré, transformées en fonction aldéhyde ou acide par oxydation dans les conditions usuelles connues de l'homme de métier telles que par exemple par action de l'oxyde de manganèse pour obtenir les aldéhydes ou du réactif de Jones pour accéder aux acides.
- Les éventuelles fonctions nitrile des produits décrits ci-dessus peuvent être, si désiré, transformées en tétrazolyle dans les conditions usuelles connues de l'homme de métier telles que par exemple par cycloaddition d'un azidure métallique tel que par exemple un azidure de trialkylétain sur la fonction nitrile ainsi qu'il est indiqué dans la méthode décrite dans l'article référencé comme suit : J. Organometallic Chemistry., 33, 337 (1971) KOZIMA S.& coll.
- Les éventuelles formes optiquement actives des produits de formule (I) peuvent être préparées par dédoublement des racémiques selon les méthodes usuelles connues de l'homme de métier.
- Les réactions de transformation de radical formyle en radical carbamoyle et de radical carbamoyle en radical nitrile sont réalisées selon les conditions usuelles connues de l'homme de métier. Ces réactions ainsi que la transformation du radical nitrile en tétrazole sont effectuées de préférence lorsque ces substituants sont portés en alpha du substituant biphényle que peut représenter -Y.

Les composés de formule (I) tels que définis ci-dessus ainsi que leurs sels d'addition avec les acides présentent d'intéressantes propriétés pharmacologiques.

Les produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes.

Certains produits de la présente invention possèdent également des propriétés antagonistes pour le récepteur à l'endothéline et sont ainsi notamment antagonistes de l'effet vasoconstricteur de l'endothéline.

Les composés de formule (I) possèdent également la propriété d'améliorer les fonctions cognitives.

Ces propriétés justifient leur application en thérapeutique et l'invention a également pour objet à titre de médicaments, les produits tels que définis par la formule (I) ci-dessus, lesdits produits de formule (I) étant sous toutes les formes isoméres possibles racémiques ou optiquement actives, ainsi que les sels d'addition avec les acides minéraux ou organiques pharmaceutiquement acceptables desdits produits de formule (I).

L'invention a plus particulièrement pour objet, à titre de médicaments, les produits décrits ci-aprés dans les exemples et notamment les produits de formule (I) suivants :
- l'acide 2-butyl 3-[(2'-1H-tétrazol 5-yl) (1,1'-biphényl) 4-yl) méthyl] pyrazolo-(1,5-a)pyridine 4-carboxylique,
- l'acide 4'-[(2-butyl pyrazolo-(1,5-a)pyridin 3-yl) méthyl] (1,1'-biphényl) 2-carboxylique,
- l'acide 4'-[(2-butyl imidazo-(1,2-a)pyridin 3-yl) méthyl] (1,1'-biphényl) 2-carboxylique,
- l'acide 4-[(2-butyl imidazo-(1,2-a)pyridin 3-yl) méthyl] benzoïque,
ainsi que leurs sels d'addition avec les acides minéraux ou organiques pharmaceutiquement acceptables.

Les médicaments, objet de l'invention, peuvent être utilisés dans le traitement des affections cardiovasculaires présentant une altération de la vasomotricité : infarctus du myocarde, insuffisance cardiaque, insuffisance rénale, angine de poitrine, spasme vasculaire cérébral, maladie de Raynaud, hypertension artérielle et toutes les affections consécutives à une ischémie. Ces médicaments, objet de l'invention, pourraient également être utilisés pour le traitement de l'athérosclérose, de l'asthme et de différents types de spasmes viscéraux, ainsi qu'à titre de substances protectrices neuronales ou encore dans la prévention des resténoses post-angioplastie ou encore du glaucome.

Ils peuvent également être utilisés dans le traitement de certains désordres gastro-intestinaux, gynécologiques et en particulier pour un effet relaxant au niveau de l'utérus.

Les médicaments, objet de l'invention peuvent également être utilisés dans le traitement des troubles de la mémoire, de la démence sénile et de la maladie d'Alzheimer.

L'invention s'étend aux compositions pharmaceutiques renfermant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Les composés de départ de formules (II), (III), (VI) et (IX) peuvent être disponibles dans le commerce ou peuvent être préparés selon les méthodes usuelles connues de l'homme de métier.

Parmi les composés de formule (II) qui peuvent être trouvés dans le commerce, on peut citer par exemple le méta-aminobenzoate de méthyle que l'on peut trouver, par exemple, sous forme de produit commercialisé par exemple par LANCASTER.

On peut également citer par exemple l'orthonitroaniline que l'on peut trouver, par exemple, sous forme de produit commercialisé par UCB.

Les composés de formule (VI) peuvent constituer des halocétones et notamment des bromocétones.

Parmi les exemples de préparation de tels composés de formule (VI) décrits dans la littérature, on peut citer notamment les références suivantes :
J. Hel. Chem. 20, p. 623-628 (1983)
Liebigs Ann. Chem. 697, p. 62-68 (1966).

Les composés de formule (III) peuvent constituer notamment des dérivés de l'acétylène.

Parmi les exemples de préparation de tels composés de formule (III) décrits dans la littérature, on peut citer notamment les références suivantes :
J. Am. Chem. Soc. 1937, 59, p. 1490.
Synthesis 1977, p. 777.

Certains produits de formule (IX) telle que définie ci-dessus non disponibles dans le commerce peuvent être préparés en soumettant le composé de formule (IXₐ) : 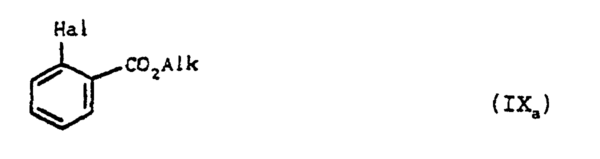 dans laquelle Alk et Hal sont tels que définis ci-dessus, à l'action d'un composé de formule (IX_{b}) : 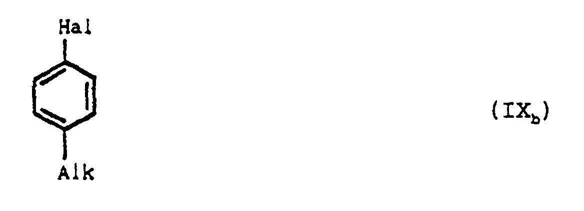 dans laquelle Alk et Hal ont la signification indiquée précédemment, la réaction se réalisant par exemple en présence de cuivre en poudre à une température d'environ 100°C à 300°C, pour obtenir un produit de formule (IX_{c}) : 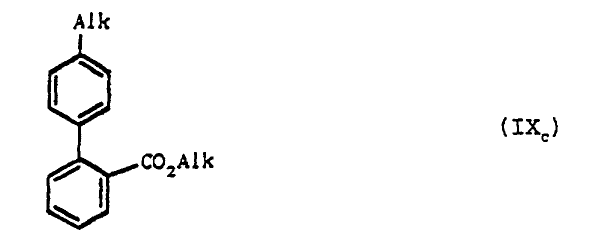 dans laquelle Alk ont les significations indiquées précédemment, dont le radical carboxy estérifié peut, si désiré, être soumis à des réactions diverses connues de l'homme de métier et notamment être libéré du radical alkyle par les méthodes classiques connues de l'homme de métier ou indiquées ci-dessus, par exemple d'hydrolyse acide ou alcaline, être transformé en radical cyano, tétrazole, arylalkyle, selon les méthodes usuelles connues de l'homme de métier et notamment telles que décrites ci-dessus ou dans la partie expérimentale, que l'on peut soumettre à une réaction de bromation sur le radical alkyle par les méthodes classiques connues de l'homme de métier par exemple par action du n-bromosuccinimide dans le tétrachlorure de carbone et à la préparation de l'organo métallique correspondant et notamment bromozincique pour obtenir un composé correspondant à la formule BrZnR₅Y' (IX).

Des exemples de préparation de composés de formule (IX) sont décrits dans la littérature et des exemples en sont donnés notamment dans le brevet US 4,880,804 ou par exemple dans la référence Chemistry and Industry 7 september 1987 HOWARD and COLQUHOUN pp. 612-617.

La présente invention a enfin pour objet à titre de produits industriels nouveaux et notamment à titre de produits intermédiaires nécessaire à la préparation des produits de formule (I), les composés de formules (IV), (V), (VIII), (IX) et (X).

Les exemples suivants illustrent l'invention sans toutefois la limiter.

### EXEMPLE 1 : (4-bromophényl) (2-butyl pyrazolo(1,5-a) pyridin-3-yl) méthanone

### Stade A : 1-(4-bromophényl) 2-heptyn-1-one

On agite à température ambiante pendant environ 2 heures 30 :
- 49 cm³ de 1-hexyne
- 75 cm³ de triéthylamine
- 40 mg d'iodure cuivreux
- 40 mg de bis triphénylphosphine palladium dichlorure
   et
- 9 g de chlorure de parabromobenzoyle.

On reprend le mélange réactionnel à l'eau et l'acétate d'éthyle, filtre et extrait à l'acétate d'éthyle.

On sèche, filtre et élimine les solvants sous pression réduite à une température d'environ 50°C.

Après chromatographie sur silice (essence G : 90/acétate d'éthyle : 10), on obtient 7,63 g de produit attendu.

### Analyses

Spectre IR (CDCl₃)
- C ≡ C - 2245 cm⁻¹
   2230 cm⁻¹
   2205 cm⁻¹
- C = 0 1643 cm⁻¹
   aromatiques 1585 cm⁻¹ - 1570 cm⁻¹
   1482 cm⁻¹

### Stade B : (4-bromophényl) (2-butyl pyrazolo(1,5-a) pyridin-3-yl) méthanone

On agite 15 minutes un mélange de :
- 2,5 g de mésitylènesulfonate de N-aminopyridinium (préparé selon Synthesis 1977 page 1 Y. TAMURA, J. MINOMIKAWA, M. IKEDA)
- 25 cm³ d'acétonitrile
   et
- 3,5 g de carbonate de potassium,
à la température ambiante, puis on ajoute :
- 2,27 g du produit obtenu au stade A, en solution dans 25 cm³ d'acétonitrile.

On agite 24 heures, à température ambiante puis chauffe à reflux pendant 3 heures.

Après chromatographie sur silice (essence G : 80/acétate d'éthyle : 20), on filtre, sèche, empâte à l'éther, filtre, sèche sous pression réduite et obtient 1,9 g de produit attendu.
F = 84°C

| Analyses pour C₁₈H₁₇BrN₂O = 357,26 | | | | | |
|---|---|---|---|---|---|
| Microanalyse | | | | | |
| | C | H | Br | N | O |
| % calculés | 60,52 | 4,80 | 22,37 | 7,84 | 4,48 |
| % trouvés | 60,7 | 4,8 | 22,1 | 7,8 | |

Spectre IR (CHCl₃)
- C = O 1638 cm⁻¹
   aromatiques et hétéroaromatiques 1620 cm⁻¹ - 1589 cm⁻¹
   1567 cm⁻¹ - 1539 cm⁻¹
   1509 cm⁻¹

### EXEMPLE 2 : 4-[(2-butyl pyrazolo(1,5-a) pyridin-3-yl) carbonyl benzonitrile

On chauffe 24 heures, au reflux :
- 1,5 g du produit obtenu à l'exemple 1
- 100 cm³ de diméthylformamide
   et
- 3 g de cyanure cuivreux.

On reprend le mélange réactionnel par 400 cm³ d'eau et 400 cm³ d'acétate d'éthyle, filtre et extrait à l'acétate d'éthyle.

On sèche, filtre et élimine les solvants sous pression réduite à une température d'environ 60°C.

Après chromatographie sur silice (essence G : 80/acétate d'éthyle : 20) on obtient 1,03 g de produit attendu.
F = 116°C

### Analyses

Spectre IR (CDCl₃)
- C ≡ N 2230 cm⁻¹
- C = O 1635 cm⁻¹
   aromatiques et hétéroaromatiques 1560 cm⁻¹ - 1540 cm⁻¹
   1508 cm⁻¹

### EXEMPLE 3 : acide 4-[(2-butyl pyrazolo(1,5-a) pyridin-3-yl) carbonyl] benzoïque

On chauffe 2 heures, au reflux :
- 845 mg du produit obtenu à l'exemple 2
   et
- 15 cm³ d'un mélange en proportions égales d'acide sulfurique, acide acétique et eau.

On refroidit, reprend à l'eau, filtre, lave à l'eau et sèche à 80°C, sous pression réduite.

On dissout le produit obtenu dans de l'acétonitrile au reflux, filtre à chaud, concentre, glace, filtre et sèche à 80°C, sous pression réduite.
On obtient 850 mg de produit attendu. F = 196°C

| Analyses pour C₁₉H₁₈N₂O₃ = 322,37 | | | |
|---|---|---|---|
| Microanalyse : | | | |

| | C | H | N |
|---|---|---|---|
| % calculés | 70,79 | 5,63 | 8,69 |
| % trouvés | 71,0 | 5,7 | 8,4 |

Spectre IR (CDCl₃)
- C = O 1708 cm⁻¹
   1628 cm⁻¹
   aromatique 1610 cm⁻¹ - 1595 cm⁻¹
   1570 cm⁻¹ - 1540 cm⁻¹
   1504 cm⁻¹

### EXEMPLE 4 : acide 4-[(2-butyl 4,5,6,7-tétrahydro pyrazolo(1,5-a) pyridin-3-yl) méthyl] benzoïque

On hydrogène à la température ambiante pendant environ 30 minutes.
- 437 mg du produit obtenu à l'exemple 3 dans 25 cm³ d'acide acétique et 5 cm³ d'acide perchlorique en présence de 200 mg de Palladium à 10 % sur charbon actif.

On filtre, lave à l'acétate d'éthyle, ajoute 100 cm³ d'eau, décante, extrait à l'acétate d'éthyle, sèche, filtre et élimine les solvants à 50°C sous pression réduite.

On dissout le résidu dans 50 cm³ de soude 1N et 20 cm³ d'éther, agite, décante et extrait par 2 x 20 cm³ de soude 1N.

On acidifie par de l'acide chlorhydrique concentré et extrait par du chloroforme à 20 % de méthanol puis sèche, filtre et élimine les solvants à 50°C sous pression réduite.

On empâte à l'éther, filtre et sèche à 60°C sous pression réduite.

On obtient le produit purifié par dissolution dans de l'acétonitrile en opérant ainsi qu'il est indiqué pour l'exemple 3. On obtient 260 mg de produit attendu. F = 148-150°C

| Analyses pour C₁₉H₂₄N₂O₂ = 312,42 | | | |
|---|---|---|---|
| Microanalyse : | | | |
| | C | H | N |
| % calculés | 73,05 | 7,74 | 8,97 |
| % trouvés | 73,3 | 7,8 | 9,0 |

Spectre IR (CDCl₃)
absence de cétone conjuguée
- C = O 1725 cm⁻¹ (eq)
   1892 cm⁻¹ (max)
   hétérocycle et aromatique 1610 cm⁻¹ - 1575 cm⁻¹
   1560 cm⁻¹ - 1510 cm⁻¹
   1478 cm⁻¹

### EXEMPLE 5 : 3-[(4-bromophényl) méthyl] 2-butyl pyrazolo(1,5-a) pyridine

On met en suspension :
- 1,25 g d'hydrure de lithium et d'aluminium dans 100 cm³ d'éther sulfurique puis ajoute 1,46 g de chlorure d'aluminium.

On introduit ensuite à la température ambiante 3,7 g du produit obtenu à l'exemple 1 et laisse sous agitation à la température ambiante pendant environ 2 heures.

On introduit à une température d'environ 0 à 10°C du tétrahydrofuranne à 20 % d'eau, reprend à l'eau, filtre, décante et extrait à l'acétate d'éthyle.

On sèche la phase organique, filtre et élimine les solvants sous pression réduite à une température d'environ 50°C et obtient 3,7 g de produit attendu.
F < 50°C

### Analyses

Spectre IR (CHCl₃)
système conjugué et aromatiques 1636 cm⁻¹ - 1542 cm⁻¹
1488 cm⁻¹

### EXEMPLE 6 : 4'-[(2-butyl pyrazolo(1,5-a) pyridin-3-yl) méthyl] - (1,1'-biphényl) 2-carboxylate de 1,1-diméthyléthyle

### Stade A : 3-[(4-(tributylétain) phényl) méthyl] 2-butyl pyrazolo(1,5-a) pyridine

On introduit :
- 3,13 g du produit obtenu à l'exemple 5
- 30 cm³ de diméthylformamide
- 13,8 cm³ de bis tributylétain
   et
   - 640 mg de bis (triphénylphosphine palladium)dichlorure (FLUKA).

On chauffe à 120°C pendant 30 minutes. On reprend à l'eau, extrait à l'acétate d'éthyle et élimine les solvants sous pression réduite, purifie le résidu obtenu par chromatographie sur silice (élution essence G : 80/acétate d'éthyle : 20), pour obtenir 2,6 g de produit attendu.

### Stade B : 4'-[(2-butyl pyrazolo(1,5-a) pyridin-3-yl) méthyl] (1,1'-biphényl) 2-carboxylate de 1,1-diméthyléthyle

On introduit :
- 2,6 g du produit obtenu au stade A
- 1,52 g de iodure de 2-benzoate de 1,1-diméthyléthyle
- 30 cm³ de toluène
   et
- 350 mg de bis (triphénylphosphine palladium) dichlorure.

On chauffe 3 heures au reflux.

On filtre et chasse le solvant sous pression réduite.

Après chromatographie sur silice (d'abord dans du chlorure de méthylène puis dans le mélange essence G : 80/acétate d'éthyle : 20), on obtient 1,055 g de produit attendu.
Spectre IR (CHCl₃)
- C - Otbu 1703 cm⁻¹
   O 1368 cm⁻¹
aromatiques et hétéroaromatiques 1636 cm⁻¹ - 1598 cm⁻¹
1540 cm⁻¹ - 1492 cm⁻¹

### EXEMPLE 7 : acide 4'-[(2-butyl pyrazolo(1,5-a) pyridin-3-yl) méthyl] (1,1'-biphényl) 2-carboxylique

On agite 4 heures à température ambiante :
- 1 g du produit obtenu à l'exemple 6
- 20 cm³ de chlorure de méthylène
   et
- 4 cm³ d'acide trifluoroacétique

On élimine solvant et réactif à 30°C, sous pression réduite, reprend par un mélange de 40 cm³ de soude 1N et de 20 cm³ d'éther et extrait à la soude 1N.

On acidifie la phase aqueuse par de l'acide chlorhydrique concentré et extrait à l'éther après saturation par du chlorure de sodium.

On sèche, filtre et élimine le solvant à 50°C sous pression réduite.

En opérant ainsi qu'il est indiqué pour l'exemple 3 mais en purifiant dans de l'éther isopropylique, on obtient 620 mg de produit attendu. F = 163°C.

| Analyses pour C₂₅H₂₄N₂O₂ = 384,48 | | | |
|---|---|---|---|
| Microanalyse : | | | |
| | C | H | N |
| % calculés | 78,10 | 6,29 | 7,29 |
| % trouvés | 78,0 | 6,3 | 7,3 |

Spectre IR (CDCl₃)
OH - 3510 cm⁻¹
- C = O 1730 cm⁻¹
1697 cm⁻¹
aromatiques et hétérocycles 1637 cm⁻¹ 1612 cm⁻¹
1600 cm⁻¹ 1575 cm⁻¹
1565 cm⁻¹ 1543 cm⁻¹
1515 cm⁻¹ 1494 cm⁻¹
1482 cm⁻¹

### EXEMPLE 8 : 4'-[(2-butyl 4,5,6,7-tétrahydro pyrazola(1,5-a) pyridin-3-yl) méthyl] (1,1'-biphényl) 2-carboxylate de 1,1-diméthyléthyle

On agite sous hydrogène à la température ambiante pendant quelques minutes jusqu'à fin d'absorption.
- 220 g du produit obtenu à l'exemple 6
- 20 cm³ d'acide acétique
   et
- 100 mg de dioxyde de platine à 82 %.

On filtre et extrait à l'acétate d'éthyle. On purifie par chromatographie sur silice (essence G : 80/acétate d'éthyle : 20) et obtient 145 mg de produit attendu.

### Analyses

Spectre IR (CHCl₃)
- C = O 1704 cm⁻¹
   système conjugué et aromatiques 1612 cm⁻¹ - 1600 cm⁻¹
   1562 cm⁻¹ - 1509 cm⁻¹

### EXEMPLE 9 : acide 4'-[(2-butyl 4,5,6,7-tétrahydro-pyrazolo-(1,5-a) pyridin-3-yl) méthyl] (1,1'-biphényl) 2-carboxylique

On agite 6 heures à température ambiante :
- 118 mg du produit obtenu à l'exemple 8
- 2 cm³ de chlorure de méthylène
   et
- 0,5 cm³ d'acide trifluoroacétique.

On élimine solvant et réactif sous pression réduite, reprend par un mélange de 50 cm³ d'éther et de 50 cm³ de soude 1N, décante et acidifie la phase aqueuse par addition d'acide chlorhydrique et extrait par une solution de chloroforme à 20 % de méthanol.

On empâte avec de l'acétonitrile, filtre, sèche à 80°C sous pression et obtient 80 mg de produit attendu. F = 162°C.

| Analyses | | | |
|---|---|---|---|
| Microanalyse pour C₂₅H₂₈N₂O₂ = 388,51 | | | |

| | C | H | N |
|---|---|---|---|
| % calculés | 77,29 | 7,26 | 7,21 |
| % trouvés | 77,5 | 7,4 | 7,1 |

### EXEMPLE 10 : 2-butyl 3-(4-bromobenzoyl) pyrazolo(1,5-a) pyridine 6-carboxylate d'éthyle

On agite 3 heures à température ambiante :
- 13,8 g de 2,4,6-triméthylbenzènesulfonate de 1-aminopyridinium 3-carboxylate d'éthyle (composé A) (préparé selon :
   Synthesis 1977 page 1 Y. TAMURA, J. MINOMIKAWA, M. IKEDA)
- 150 cm³ de diméthylformamide
- 5,7 g de carbonate de potassium
   et
- 10 g du produit obtenu au stade A de l'exemple 1 puis introduit :

- 6,9 g de composé A
   et
- 2,85 g de carbonate de potassium et poursuit l'agitation 2 heures puis introduit à nouveau :

- 6,9 g de composé A
   et
- 2,85 g de carbonate de potassium et agite encore 2 heures à température ambiante.

On reprend à l'eau et extrait à l'acétate d'éthyle.

On sépare par chromatographie sur silice (essence G : 80/acétate d'éthyle : 20) et obtient 7,38 g de produit attendu (F ≤ 50°C) et 8,25 g de l'isomère correspondant qui constitue l'exemple 16.

### Analyses

Spectre IR (CHCl₃)
- C = O 1721 cm⁻¹
   1633 cm⁻¹
   aromatiques et hétéroaromatiques 1587 cm⁻¹ - 1565 cm⁻¹
   1541 cm⁻¹ - 1519 cm⁻¹

### EXEMPLE 11 : acide 2-butyl 3-[(4-bromophényl) méthyl] pyrazolo(1,5-a) pyridin 6-carboxylique

On mélange :
- 6,6 g du produit obtenu à l'exemple 10
- 300 cm³ d'éthylène glycol
- 60 cm³ d'une solution d'hydrate d'hydrazine à 64 %
   et
- 10 cm³ de lessive de potasse
   et chauffe pendant environ 2 heures à une température d'environ 140°C puis pendant environ 2 heures en distillant l'eau du mélange réactionnel.

On refroidit, verse la solution dans 600 cm³ d'eau et de glace, acidifie par addition d'acide chlorhydrique concentré, agite à 0°C, filtre, sèche sous pression réduite à la température ambiante et obtient 4 g de produit attendu.

### EXEMPLE 12 : 2-butyl 3-[(4-bromophényl) méthyl] pyrazolo(1,5-a) pyridin 6-carboxylate de méthyle

A une suspension de 4 g du produit obtenu à l'exemple 11 dans 100 cm³ de chlorure de méthylène on ajoute à température ambiante 100 cm³ d'une solution de diazométhane dans le chlorure de méthylène.

On élimine le solvant à 40°C, sous pression réduite.

On chromatographie sur silice (Hexane : 80/acétate d'éthyle : 20) et obtient 3,7 g de produit attendu.
F < ou = 50°C.
Spectre IR (CHCl₃)
- C = O 1721 cm⁻¹
   système conjugué et hétéroaromatiques 1635 cm⁻¹ - 1530 cm⁻¹

### EXEMPLE 13 : 2-butyl 3-[(2'-cyano (1,1'-biphényl) 4-yl) méthyl] pyrazolo(1,5-a) pyridin 6-carboxylate de méthyle

### Stade A : 2-butyl 3-[(4-(tributylétainphényl) méthyl] pyrazolo (1,5-a) pyridine) 6-carboxylate de méthyle

On introduit :
- 3,7 g du produit obtenu à l'exemple 12
- 35 cm³ de diméthylformamide
- 13,2 cm³ d'hexabutyldistannane (FLUKA)
   et
- 620 mg de chlorure de bis(triphénylphosphine) palladium.

On chauffe à 120°C pendant 1 heure puis abandonne 30 minutes à température ambiante.

On reprend à l'eau et extrait à l'acétate d'éthyle, sèche et évapore à sec.

On chromatographie le résidu sur silice (Hexane : 80/acétate d'éthyle : 20) et obtient 2,9 g de produit attendu. Stade B : 2-butyl 3-[(2'-cyano (1,1'-biphényl) 4-yl) méthyl] pyrazolo(1,5-a) pyridin 6-carboxylate de méthyle

On introduit :
- 2,9 g du produit obtenu au stade A
- 950 mg de 2-bromobenzonitrile
- 50 cm³ de toluène
   et
- 330 mg de chlorure de bis(triphénylphosphine) palladium.

On chauffe au reflux 6 heures puis abandonne 16 heures à température ambiante.

On filtre et élimine le solvant sous pression réduite.

On chromatographie le résidu sur silice (Hexane : 80/acétate d'éthyle : 20), élimine le solvant sous pression réduite et obtient 1,625 g de produit attendu.
Spectre IR (CHCl₃)
- C ≡ N 2226 cm⁻¹
- CO₂Me 1720 cm⁻¹ - 1437 cm⁻¹
   aromatiques et hétéroaromatiques 1635 cm⁻¹ - 1613 cm⁻¹
   1598 cm⁻¹ - 1533 cm⁻¹

### EXEMPLE 14 : 2-butyl 3-[(2'-(1H-tétrazol-5-yl) (1,1'-biphényl) 4-yl) méthyl] pyrazolo(1,5-a) pyridin 6-carboxylate de méthyle

On introduit :
- 1,625 g du produit obtenu à l'exemple 13
- 20 cm³ de toluène
   et
- 800 mg d'azidure de triméthylétain
et chauffe au reflux pendant environ 72 heures au cours desquelles on ajoute au bout d'environ 16 heures et 48 heures chaque fois 800 mg d'azidure de triméthylétain.

On ajoute 20 cm³ de tétrahydrofuranne puis effectue un barbotage de gaz chlorhydrique pendant 30 minutes à température ambiante puis élimine le gaz chlorhydrique par barbotage d'azote et évapore le solvant sous pression réduite.

On chromatographie sur silice (chloroforme : 90/méthanol : 10), on élimine les solvants sous pression réduite et obtient 1,35 g de produit attendu.

### Analyses

Spectre IR (chloroforme)
= C - NH - 3408 cm⁻¹
- C = O 1721 cm⁻¹
- C = O 1721 cm⁻¹
(CO₂Me) 1437 cm⁻¹
aromatiques et hétéroaromatiques 1635 cm⁻¹ - 1604 cm⁻¹
1579 cm⁻¹ - 1543 cm⁻¹
1534 cm⁻¹ - 1515 cm⁻¹

### EXEMPLE 15 : acide 2-butyl 3[(2'-(lH-tétrazol-5-yl) (1,1'-biphényl) 4-yl) méthyl] pyrazolo(1,5-a) pyridin 6-carboxylique

On chauffe au reflux pendant 2 heures :
- 1,65 g du produit obtenu à l'exemple 14
- 80 cm³ d'alcool terbutylique
   et
- 40 cm³ de lessive de potasse

On élimine le terbutanol sous pression réduite, dilue par environ 250 cm³ d'eau, filtre et extrait à l'éther.

On acidifie la phase aqueuse par addition d'acide chlorhydrique concentré sous agitation à une température d'environ 0 à 5°C et abandonne 16 heures à température ambiante puis filtre et sèche sous pression réduite.

On dissout le produit obtenu dans 300 cm³ d'un mélange eau : 50/isopropanol : 50 au reflux, filtre, élimine l'isopropanol sous pression réduite, glace, essore, sèche et obtient 1 g de produit attendu.

### Analyses

Spectre IR (NUJOL)
- C = O 1697 cm⁻¹
   système conjugué 1636 cm⁻¹ - 1603 cm⁻¹
   1534 cm⁻¹ - 1485 cm⁻¹

### EXEMPLE 16 : 2-butyl 3-(4-bromobenzoyl) pyrazolo(1,5-a) pyridine 4-carboxylate d'éthyle

Ce produit est l'isomère du produit obtenu à l'exemple 10, après séparation par chromatographie, on obtient 8,25 g de produit attendu. F < 50°C

### Analyses

Spectre IR (CHCl₃)
- C = O 1720 cm⁻¹
   1652 cm⁻¹
   aromatiques et hétéroaromatiques 1624 cm⁻¹ - 1586 cm⁻¹
   1569 cm⁻¹ - 1550 cm⁻¹
   1518 cm⁻¹

### EXEMPLE 17 : acide 2-butyl 3-[(4-bromophényl) méthyl] pyrazolo(1,5-a) pyridin 4-carboxylique

On opère comme à l'exemple 11 à partir de :
- 6,75 g du produit obtenu à l'exemple 16 en utilisant
- 200 cm³ d'éthylène glycol
- 70 cm³ d'une solution d'hydrate d'hydrazine à 64 %
   et
- 10 cm³ de lessive de potasse
on obtient 11,16 g de produit attendu, utilisé tel quel pour l'exemple suivant.

### EXEMPLE 18 : 2-butyl 3-[(4-bromophényl) méthyl] pyrazolo(1,5-a) pyridin 4-carboxylate de méthyle

On opère comme à l'exemple 12 à partir de 11,16 g du produit obtenu à l'exemple 17.

Après chromatographie sur silice (Hexane : 80/acétate d'éthyle : 20), on obtient 3,7 g de produit attendu.
F = 80-85°C
Spectre IR (CHCl₃)
- CO₂Me 1723 cm⁻¹ et 1439 cm⁻¹
   système conjugué et hétéroaromatiques 1622 cm⁻¹ - 1590 cm⁻¹
   1560 cm⁻¹ - 1544 cm⁻¹

### EXEMPLE 19 : 2-butyl 3-[(2'-cyano (1,1'-biphényl) 4-yl) méthyl] pyrazolo(1,5-a) pyridine 4-carboxylate de méthyle

### Stade A : 2-butyl 3-[(4-(tributylétainphényl) méthyl] pyrazolo(1,5-a) pyridine 4-carboxylate de méthyle

On opère comme au stade A de l'exemple 13 à partir de :
- 4,5 g du produit obtenu à l'exemple 18 en utilisant
- 45 cm³ de diméthylformamide
- 16,5 cm³ d'hexabutyldistannane
   et
- 770 mg de chlorure de bis(triphénylphosphine) palladium on obtient 4,3 g de produit attendu.

### Stade B : 2-butyl 3-[(2'-cyano (1,1'-biphényl) 4-yl) méthyl] pyrazolo(1,5-a) pyridine 4-carboxylate de méthyle

On opère comme au stade B de l'exemple 13 en utilisant :
- 4,3 g du produit obtenu au stade A avec
- 1,3 g de 2-bromobenzonitrile
- 75 cm³ de toluène
   et
- 490 mg de chlorure de bis(triphénylphosphine) palladium on obtient 2,38 g de produit attendu. F = 92-95°C
Spectre IR (CHCl₃)
- C ≡ N 2225 cm⁻¹ (F)
- CO₂Me 1722 cm⁻¹ - 1439 cm⁻¹
   aromatiques et hétéroaromatiques 1622 cm⁻¹ - 1612 cm⁻¹
   1595 cm⁻¹ - 1570 cm⁻¹
   1555 cm⁻¹

### EXEMPLE 20 : 2-butyl 3-[(2'-(1H-tétrazol-5-yl) (1,1'-biphényl) 4-yl) méthyl] pyrazolo(1,5-a) pyridin 4-carboxylate de méthyle

On opère comme à l'exemple 14 à partir de :
- 945 mg du produit obtenu à l'exemple 19 en utilisant
- 10 cm³ de toluène
   et
- 460 mg d'azidure de triméthylétain

Après chromatographie (chloroforme : 80/méthanol : 20), on obtient 1,02 g de produit attendu.

### EXEMPLE 21 : acide 2-butyl 3-[(2'-(1H-tétrazol-5-yl) (1,1'-biphényl) 4-yl) méthyl] pyrazolo(1,5-a) pyridin 4-carboxylique

On opère comme à l'exemple 15 à partir de :
- 1,02 g du produit obtenu à l'exemple 20 en utilisant
- 50 cm³ d'alcool terbutylique
   et
- 25 cm³ de lessive de potasse

On obtient après recristallisation dans l'acétonitrile 686 mg de produit attendu. F = 214°C

| Analyses | | | |
|---|---|---|---|
| Microanalyse pour C₂₆H₂₄N₆O₂ = 452,52 | | | |

| | C | H | N |
|---|---|---|---|
| % calculés | 69,01 | 5,35 | 18,57 |
| % trouvés | 69,0 | 5,2 | 18,6 |

Spectre IR (NUJOL)
- C = O 1710 cm⁻¹
   système conjugué et aromatiques 1618 cm⁻¹ - 1606 cm⁻¹
   1580 cm⁻¹ - 1572 cm⁻¹
   1548 cm⁻¹ - 1530 cm⁻¹
   1496 cm⁻¹

### EXEMPLE 22 : 3-[(4-cyanophényl) méthyl] 2-butyl imidazo(1,2-a) pyridine

### Stade A : 1-bromo 2-hexanol

On introduit :
- 8 cm³ de 1,2 époxy hexane
   et
- 80 cm³ d'acétonitrile
refroidit à 0°C et ajoute 5,77 g de bromure de lithium.

On ajoute à 0°C, 8,43 cm³ de triméthylchlorosilane, agite 4 heures à 0°C, filtre, lave à l'acétate d'éthyle, verse sur 150 cm³ d'acide chlorhydrique 1N et agite 15 minutes.

Après chromatographie (chlorure de méthylène : 30/hexane : 70), on lave la phase organique par de l'eau saturée en chlorure de sodium, sèche, filtre, distille et obtient 10,22 g du produit attendu sous forme d'une huile incolore.

### Stade B : 1-bromo 2-hexanone

On introduit :
- 30 g du produit obtenu au stade A ci-dessus et - 120 cm³ d'acétone anhydre,
   refroidit à 0°C et en maintenant cette température : ajoute goutte à goutte 49,5 cm³ de réactif de Jones et agite 1 heure 30.

On verse sur la solution revenue à la température ambiante une solution aqueuse saturée en chlorure de sodium, extrait à l'acétate d'éthyle, lave la phase organique à l'eau, sèche, filtre, distille et obtient après chromatographie (chlorure de méthylène) 13,8 g du produit attendu sous forme d'une huile incolore.

### Analyses

Spectre IR (NUJOL)
- C = O 1730 cm⁻¹
- C = O 1714 cm⁻¹

### Stade C : 2-butyl imidazo(1,2-a) pyridine

On introduit :
- 6 g de 2-amino pyridine
   et
- 60 cm³ d'acétone anhydre,
ajoute à la température ambiante
- 16 g du produit obtenu au stade B ci-dessus
   et
- 20 cm³ d'acétone,
porte au reflux 1 heure et distille partiellement l'acétone.

On laisse revenir à la température ambiante, glace, essore, lave à l'éther éthylique, dissout l'insoluble dans une solution saturée de carbonate de sodium, extrait à l'acétate d'éthyle, lave par de l'eau saturée en chlorure de sodium, sèche, filtre et obtient 5,9 g du produit attendu sous forme d'une huile jaune.

### Analyses

Spectre IR (CHCl₃)
système conjugué et aromatiques 1638 cm⁻¹ - 1610 cm⁻¹
1492 cm⁻¹

### Stade D : 3-bromo 2-butyl imidazo(1,2-a) pyridine

On introduit :
- 3,5 g du produit obtenu au stade c) ci-dessus
   et
- 50 cm³ de chloroforme,
refroidit la solution à 10°C, ajoute 4,55 g de N-bromo succinimide et agite à cette température 30 minutes.

On verse dans une solution saturée de carbonate de sodium, extrait à l'acétate d'éthyle, lave avec une solution aqueuse saturée en chlorure de sodium, sèche, filtre et évapore à sec, après chromatographie sur silice (chlorure de méthylène : 95/méthanol : 5), on obtient 3,8 g du produit attendu sous forme d'une huile noire.

### Analyses

Spectre IR (CHCl₃)
hétérocycles 1632 cm⁻¹ - 1603 cm⁻¹
1529 cm⁻¹ - 1499 cm⁻¹

### Stade E : 4-[(2-butyl imidazo(1,2-a) pyridine 3-yl) méthyl] benzonitrile

On introduit :
- 2 g du produit obtenu au stade d) ci-dessus
- 100 cm³ de tétrahydrofuranne,
   et
- 1 g de [tétra-bis(triphénylphosphine)] palladium
ajoute à la température ambiante 35,9 cm³ de zincique de 4-bromométhyl benzonitrile et porte au reflux 1 heure.

On laisse revenir à température ambiante, verse dans l'eau, extrait à l'acétate d'éthyle, lave avec une solution aqueuse saturée en chlorure de sodium, sèche, filtre, amène à sec, après chromatographie sur silice (acétate d'éthyle), on obtient 1,1 g du produit attendu.

### Analyses

Spectre IR (CHCl₃)
- C ≡ N 2232 cm⁻¹
   système conjugué et aromatiques 1636 cm⁻¹ - 1608 cm⁻¹
   1560 cm⁻¹ - 1504 cm⁻¹

### EXEMPLE 23 : chlorhydrate de l'acide 4-[(2-butyl imidazo(1,2

### a) pyridin 3-yl) méthyl] benzoïque

### a) acide 4-[(2-butyl imidazo(1,2-a) pyridin 3-yl) méthyl) benzoïque

On introduit :
- 0,270 g du produit obtenu à l'exemple 22
- 2,7 cm³ de soude,
   et
- 1 cm³ d'éthanol,
et porte au reflux pendant environ 5 heures.

On verse sur la solution revenue à la température ambiante 100 cm³ d'eau, acidifie à l'aide d'un barbotage de SO₂ jusqu'à pH = 2, extrait à l'acétate d'éthyle, sèche la phase organique, filtre et amène à sec.

Après chromatographie sur silice (chlorure de méthylène : 90/méthanol : 10), on obtient 0,51 g de produit attendu.

### b) chlorhydrate de l'acide 4-[(2-butyl imidazo(1,2-a) pyridin-3-yl) méthyl] benzoïque

On introduit :
- 0,3 g de produit obtenu au stade a) ci-dessus
- 10 cm³ d'éthanol,
   et
- 6,6 cm³ d'acide chlorhydrique 6,6N dans l'éthanol filtre, ajoute 25 cm³ d'éther sulfurique, glace, essore et sèche sous pression réduite à 80°C.

On ajoute 8 cm³ d'isopropanol, filtre, glace, essore, lave avec 2 cm³ d'isopropanol puis par de l'oxyde de diéthyle, sèche sous pression réduite à 60°C et obtient 0,0586 g de produit attendu. F = 244°C.

### Analyses

Spectre IR (NUJOL)
C = O 1715 cm⁻¹
aromatiques et hétérocycle 1614 cm⁻¹ - 1575 cm⁻¹
1508 cm⁻¹

### EXEMPLE 24 : 4'-[(2-butyl imidazo(1,2-a) pyridine 3-yl) méthyl] (1,1'-biphényl) 2-carboxylate de méthyle

On introduit :
- 0,290 g de Zinc électrolytique
- 0,41 g de [tétra-bis(triphénylphosphine)] palladium
   et
- 1,36 g de bromométhyl]-(1,1'-biphényl) 2-carboxylate de méthyle
on sèche sous pression réduite et ajoute sous atmosphère d'azote :
- 0,75 g du produit obtenu au stade D de l'exemple 22
   et
- 22 cm³ de tétrahydrofuranne
porte le mélange 1 heure à 65°C et ajoute :
- 0,3875 g de Zinc électrolytique
   et
- 1,81 g de bromométhyl] (1,1'-biphényl) 2-carboxylate de méthyle et maintient le mélange au reflux pendant 18 heures.

On verse une solution saturée de carbonate de sodium, extrait à l'acétate d'éthyle, lave par de l'eau saturée en chlorure de sodium, sèche, filtre, évapore à sec et, après chromatographie (acétate d'éthyle), on obtient 0,500 g du produit attendu.

### Analyses

Spectre IR (CHCl₃) (chloroforme)
C = O 1722 cm⁻¹
aromatiques et hétérocycle 1638 cm⁻¹ - 1600 cm⁻¹
1565 cm⁻¹ - 1508 cm⁻¹

### EXEMPLE 25 : acide 4'-[(2-butyl imidazo(1,2-a) pyridine 3-yl) méthyl] (1,1'-biphényl) 2-carboxylique

On introduit :
- 0,500 g du produit obtenu à l'exemple 24
- 1,5 cm³ d'éthanol
   et
- 1,5 cm³ de soude,
et portel heure au reflux.

On verse sur la solution revenue à la température ambiante 100 cm³ d'eau, acidifie à l'aide d'un barbotage de SO₂ jusqu'à pH = 2, extrait à l'acétate d'éthyle, sèche la phase organique, filtre et évapore à sec.

On empâte le résidu dans 15 cm³ d'acétone, ajoute 6,6 cm³ d'éthanol, filtre, essore, sèche sous pression réduite à 80°C. On obtient 0,202 g de produit attendu. F = 196°C

### Analyses

Spectre IR (NUJOL)
C = O 1674 cm⁻¹
système conjugué et aromatiques 1599 cm⁻¹ - 1574 cm⁻¹
1560 cm⁻¹ - 1504 cm⁻¹

### EXEMPLE 26 : chlorhydrate de 4-[(2-méthyl imidazo(1,2-a) pyridin 3-yl) méthyl] benzonitrile

### Stade A : 2-méthylimidazo(1,2-a) pyridine

On introduit :
- 5 g de 2-amino pyridine-
- 250 cm³ d'acétone,
ajoute à la température ambiante
- 7,25 g de 1 bromo 2-propanone-
- 20 cm³ d'acétone,
porte 30 minutes au reflux.

On opère comme au stade c) de l'exemple 22 et obtient 5,3 g du produit attendu sous forme d'une huile orangée.

### Analyses

Spectre IR (NUJOL)
système conjugué 1638 cm⁻¹ - 1602 cm⁻¹
1549 cm⁻¹ - 1509 cm⁻¹

### Stade B : 3-bromo 2-méthyl imidazo(1,2-a) pyridine

On introduit :
- 5,3 g du produit obtenu au stade a) ci-dessus-
- 150 cm³ de chloroforme,
refroidit la solution à 10°C, ajoute 7,5 g de N-bromo Succinimide et laisse agiter 30 minutes à 10°C.

On opère comme au stade d) de l'exemple 22 et obtient 7,47 g du produit attendu. F = 70°C.

### Stade C : 4-[(2-méthyl imidazo(1,2-a) pyridin 3-yl) méthyl] benzonitrile

On introduit :
- 3,9 g du produit obtenu au stade b) ci-dessus
- 24 cm³ de tétrahydrofuranne,
   et
- 2,57 g de [tétra-bis(triphénylphosphine)] palladium agite à la température ambiante et ajoute 77,8 cm³ de zincique de 4-bromométhyl benzonitrile et porte 2 heures au reflux.

On verse une solution saturée de bicarbonate de sodium, extrait à l'acétate d'éthyle, lave par de l'eau saturée en chlorure de sodium, sèche, filtre, sèche et après chromatographie (chlorure de méthylène : 95/méthanol : 5), on obtient 5 g de produit attendu.

### Stade D : chlorhydrate de 4-[(2-méthyl imidazo(1,2-a) pyridin 3-yl) méthyl] benzonitrile

On introduit :
- 5 g de produit obtenu au stade c) ci-dessus
- 80 cm³ d'acétate d'éthyle,
   et
- 3,5 cm³ d'acide chlorhydrique 6,6N dans l'éthanol glace, essore, lave par de l'acétate d'éthyle, sèche sous pression réduite à 80°C et obtient 5 g de produit attendu.
F = 230°C.

### Analyses

Spectre IR (NUJOL)
C ≡ N 2230 cm⁻¹
aromatiques et système conjugué 1638 cm⁻¹ - 1610 cm⁻¹
1570 cm⁻¹ - 1504 cm⁻¹

### EXEMPLE 27 : acide 4-[(2-méthyl imidazo(1,2-a) pyridin 3-yl) méthyl] benzoïque

On introduit :
- 2,6 g du produit obtenu à l'exemple 26
- 150 cm³ de soude
et porte au reflux pendant environ 8 heures.

On laisse revenir à la température ambiante, verse une solution aqueuse saturée de dihydrogénophosphate de sodium (pH = 4), extrait par 6 x 300 cm³ d'acétate d'éthyle, sèche la phase organique et filtre.

On ajoute 55 cm³ d'isopropanol, filtre, glace, essore, lave par 5 cm³ d'isopropanol puis par de l'éther éthylique, sèche sous pression réduite à 100°C et obtient 0,410 g de produit attendu. F = 217°C

### Analyses

Spectre IR (NUJOL)
C = O 1698 cm⁻¹
système conjugué et aromatiques 1612 cm⁻¹ - 1578 cm⁻¹
1506 cm⁻¹

### EXEMPLE 28 : (4-bromophényl) (2-butyl 7-méthyl pyrazolo(1,5-a) pyridin-3-yl) méthanone

On introduit :
- 11,4 g du produit obtenu au stade a) de l'exemple 1
- 250 cm³ de diméthylformamide
- 9,81 g de 2,4,6-triméthylbenzènesulfonate de 1-amino 2-méthylpyridinium (obtenu selon Synthesis 1977, page 1,
   Y. TAMURA, J. MINOMIKAWA, M. IKEDA)
   et
- 5,11 g de carbonate de potassium
   et agite 5 heures à température ambiante.

Puis on reprend avec 200 cm³ d'eau et 200 cm³ d'acétate d'éthyle, lave à l'eau salée et évapore à sec sous pression réduite à 40°C.

On chromatographie le résidu sur silice (essence G : 80/acétate d'éthyle : 20), empâte à l'éther, filtre, recristallise dans l'acétonitrile et obtient 6,66 g de produit attendu. F = 118°C

### Analyses

Spectre IR (CHCl₃)
- C = O 1636 cm⁻¹
   Aromatiques et hétéroaromatiques 1584 cm⁻¹ - 1554 cm⁻¹
   1516 cm⁻¹

### EXEMPLE 29 : 3-[(4-bromophényl) méthyl] 2-butyl 7-méthyl pyrazolo(1,5-a) pyridine

On introduit :
- 100 cm³ d'éther anhydre
   et
- 0,924 g d'hydrure de lithium et d'aluminium
puis ajoute lentement :
- 1,08 g de chlorure d'aluminium
et additionne goutte à goutte une solution de 3 g du produit obtenu à l'exemple 28 dans 100 cm³ d'éther et laisse sous agitation 2 heures à température ambiante.

On opère ensuite comme à l'exemple 5 et obtient 2,8 g de produit attendu. F = 54°C

### Analyses

Spectre IR (CHCl₃)
système conjugué aromatique 1639 cm⁻¹ - 1557 cm⁻¹

### EXEMPLE 30 : 4'-[(2-butyl 7-méthyl pyrazolo(1,5-a) pyridin 3-yl) méthyl] (1,1'-biphényl) 2-carbonitrile

### Stade A : 2-butyl 7-méthyl 3-[(4-(tributylétain) phényl) méthyl] pyrazolo(1,5-a) pyridine

On opère comme au stade A de l'exemple 13, en utilisant :
- 0,705 g du produit obtenu à l'exemple 29
- 8 cm³ de diméthylformamide
- 3 cm³ d'hexabutyldistannane (FLUKA)
   et
- 143 mg de chlorure de bis(triphénylphosphine) palladium.

Après chromatographie sur silice (essence G : 90/acétate d'éthyle : 10) on obtient 0,734 g de produit attendu.
Spectre IR (CHCl₃)
aromatiques et système conjugué 1640 cm⁻¹ - 1590 cm⁻¹
1558 cm⁻¹ - 1498 cm⁻¹

### Stade B : 4'-[(2-butyl 7-méthyl pyrazolo(1,5-a) pyridin 3-yl) méthyl] (1,1'-biphényl) 2-carbonitrile

On opère comme au stade B de l'exemple 13 à partir de :
- 0,734 g du produit obtenu ci-dessus en a) en utilisant
- 285 mg de 2-bromobenzonitrile
- 10 cm³ de toluène
   et
- 100 mg de chlorure de bis(triphénylphosphine) palladium.

Après chromatographie sur silice (éluant Hexane - acétate d'éthyle 80-20) on obtient 156 mg de produit attendu.
Spectre IR (CHCl₃)
- C ≡ N 2225 cm⁻¹
   aromatiques et hétéroaromatiques 1638 cm⁻¹ - 1612 cm⁻¹
   1598 cm⁻¹ - 1555 cm⁻¹
   1515 cm⁻¹ - 1500 cm⁻¹
   1477 cm⁻¹
et 74 mg de produit correspondant à l'exemple 31.

### EXEMPLE 31 : 3,3'-[[(1,1'-biphényl) 4,4'-diyl] bis-méthyl] bis 2-butyl 7-méthyl pyrazolo(1,5-a) pyridine

Le produit de l'exemple 31 est obtenu par séparation chromatographique de l'exemple 30 ci-desssus, on obtient 74 mg du produit attendu.

### Analyses

spectre IR (CHCl₃)
aromatiques et hétéroaromatiques 1638 cm⁻¹ - 1610 cm⁻¹
1555 cm⁻¹ - 1498 cm⁻¹

### EXEMPLE 32 : 2-butyl 7-méthyl 3-[[2'-(1H-tétrazol-5-yl) (1,1'-biphényl) 4-yl] méthyl] pyrazolo(1,5-a) pyridine

On opère comme à l'exemple 14.

A partir de :
- 0,258 g de produit obtenu à l'exemple 31 en utilisant
- 6 cm³ de toluène
   et
- 0,143 mg d'azoture de triméthylétain
et laisse agiter 5 jours.

On ajoute 6 cm³ de tétrahydrofuranne puis effectue un barbotage de gaz chlorhydrique pendant 30 minutes puis d'azote pendant une heure, dilue à l'eau, extrait à l'acétate d'éthyle, réunit les phases organiques, lave à l'eau salée, sèche et élimine les solvants sous pression réduite à 50°C.

On reprend dans l'acétonitrile et obtient 100 mg de produit attendu.

### Analyses

Spectre IR (chloroforme)
système conjugué et aromatique 1636 cm⁻¹ - 1606 cm⁻¹
1580 cm⁻¹ - 1508 cm⁻¹

### EXEMPLE 33 de composition pharmaceutique

On a préparé des comprimés répondant à la formule suivante :
Produit de l'exemple 21 10 mg
Excipient pour un comprimé terminé à 100 mg
(détail de l'excipient : lactose, talc, amidon, stéarate de magnésium).

### RESULTATS PHARMACOLOGIQUES

### 1 - Test sur le récepteur de l'angiotensine II

On utilise une préparation membranaire fraîche obtenue à partir de foie de rat. Le tissu est broyé au polytron dans un tampon Tris 50 mM pH 7,4, le broyage est suivi de 3 centrifugations à 30 000 g 15' avec reprises intermédiaires des culots dans le tampon Tris pH 7,4.

Les derniers culots sont remis en suspension dans un tampon d'incubation (Tris 20 mM, NaCl 135 mM, KCl 10 mM, glucose 5 mM, MgCl₂ 10 mM, PMSF 0,3 mM, bacitracine 0,1 mM, BSA 0,2 %).

On répartit des fractions aliquotes de 2 cm³ dans des tubes à hémolyse et ajoute de la ¹²⁵ I angiotensine II (25 000 DPM/tube) et le produit à étudier. (Le produit est d'abord testé à 3 x 10⁻⁵M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %).

La liaison non spécifique est déterminée par addition du produit de l'exemple 94 du brevet européen 0253310, à 10⁻⁵ M (en triple). On incube à 25°C pendant 150 minutes, remet au bain-marie à 0°C, 5 minutes, filtre sous vide, rince au tampon Tris pH 7,4 et compte la radioactivité en présence du scintillant Triton.

Le résultat est exprimé directement en concentration inhibitrice 50 % (CI₅₀), c'est-à-dire en concentration de produit étudié, exprimée en nM, nécessaire pour déplacer 50 % de la radioactivité spécifique fixée sur le récepteur étudié.

| Résultats : | |
|---|---|
| Produit de l'exemple | CI₅₀ en nanomoles |
| 21 | 0,5 |
| 32 | 24,0 |

### 2 - Test d'activité antagoniste de l'angiotensine II chez le rat démédullé

Des rats mâles Sprague-Dawley (250 à 350 g) sont anesthésiés par une injection intra-péritonéale de pentobarbital sodique (60 mg/kg). La pression artérielle diastolique est enregistrée grâce à un cathéter (PE50) hépariné introduit dans la carotide gauche de l'animal, et relié à un calculateur de pression (Gould, Pressure Processor) par l'intermédiaire d'un capteur de pression Gould.

Un cathéter est introduit dans la jugulaire droite de l'animal afin de permettre l'injection des molécules à étudier.

L'animal est placé sous respiration assistée. Une section bilatérale des nerfs vagues est effectuée. Le rat est alors démédullé.

Après une période de stabilisation suffisante, l'étude de l'antagonisme des molécules vis-à-vis de l'angiotensine II (Hypertensine, CIBA) est abordée de la façon suivante :
1 - Trois injections consécutives d'angiotensine II (0,75 microgrammes/kg) espacées de 15 minutes permettent d'obtenir une réponse pressive reproductible et stable.
2 - Tout en gardant une périodicité de 15 minutes pour l'administration d'angiotensine II, les molécules (0,01 à 10 mg/kg) sont injectées 5 minutes avant l'angiotensine II.
Les effets presseurs de l'angiotensine II en présence de l'antagoniste sont exprimés en pourcentage des effets presseurs de l'angiotensine II administrée seule. La dose inhibitrice à 50 % de l'effet étudié est ainsi déterminée (DI₅₀).
Chaque animal est considéré comme son propre témoin.

| Résultats : | |
|---|---|
| Produit de l'exemple | DI₅₀ en mg/kg |
| 21 | 0,3 |

### 3 - Test d'activité antagoniste de l'angiotensine II par voie orale chez le rat démedullé

Des rats mâles Sprague Dawley de poids homogène (300-330 g) sont sélectionnés. Les animaux sont répartis en plusieurs groupes (n ≥ 6 par groupe), recevant soit le solvant (groupe témoin) soit la molécule antagoniste de l'angiotensine II. Les doses administrées sont déterminées à partir de la DI₅₀ IV. Le solvant utilisé est la méthyl cellulose (5 ml/kg).

45 minutes après le gavage, les animaux sont anesthésiés au pentobarbital sodique (60 mg/kg, IP). La pression artérielle diastolique est enregistrée grâce à un cathéter (PE₅₀) hépariné introduit dans la carotide gauche de l'animal, et relié par l'intermédiaire d'un capteur de pression (Gould, P10 EZ) à un calculateur de pression (Gould, Pressure Processor). Le rat est mis sous respiration assistée, puis démédullé et bivagotomisé.

Deux injections d'angiotensine II (0,75 µg/kg ; Hypertensine, Ciba) sont effectuées par la veine pudendale, respectivement 60 et 75 minutes après le gavage des animaux.

Les effets presseurs de l'angiotensine II en présence de l'antagoniste sont exprimés en pourcentage des effets presseurs de l'angiotensine II administrée dans le groupe témoin.

Pour chaque produit, la dose inhibitrice à 50 % de l'effet étudié (DI₅₀) est ainsi déterminée.

| Produit de l'exemple | DI₅₀ en mg/kg |
|---|---|
| 21 | 2,35 |

## Revendications

1. a) Produits de formule (I) : 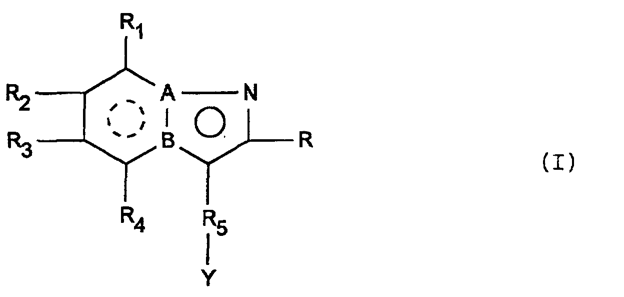 dans laquelle :
l'un de A ou B représente un atome d'azote et l'autre représente un atome de carbone tel que l'hétérobicycle ainsi formé représente un radical imidazo pyridine, pyrazolo pyridine ou pyrazolo tétrahydro imidazotétrahydropyridine, pyridine,
R représente un radical n-butyle ou méthyle,
R₁, R₂, R₃ et R₄ sont tels que trois d'entre eux représentent un atome d'hydrogène et l'autre représente un atome d'hydrogène, un radical carboxy libre ou estérifié par un radical alkyle linéaire ou ramifié renfermant au plus 4 atomes de carbone,
R₅ représente un radical méthylène ou un radical -C=O,
et Y représente le radical phényle ou biphényle substitué par un atome d'halogène, un radical cyano, carboxy libre ou estérifié par un radical alkyle ou un radical tétrazolyle, les radicaux alkyle étant linéaires ou ramifiés et renfermant au plus 4 atomes de carbone, à l'exception des produits dans lesquels R₁, R₂, R₃ et R₄ représentent un atome d'hydrogène
A représente N,
R représente un radical alkyle,
R₅ représente -CO
et Y représente le radical phényle substitué par un atome d'halogène.
b) Les produits suivants :
- (4-bromophényl) (2-butyl pyrazolo (1,5-a) pyridin-3-yl) méthanone
- (4-bromophényl) (2-butyl 7- méthyl pyrazolo (1,5-a) pyridin-3-yl) méthanone
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

2. l'acide 2-butyl 3-[(2'-1H-tétrazol 5-yl) (1,1'-biphényl) 4-yl) méthyl] pyrazolo-(1,5-a)pyridine 4-carboxylique,
- l'acide 4'-[(2-butyl pyrazolo-(1,5-a)pyridin 3-yl) méthyl] (1,1'-biphényl) 2-carboxylique,
- l'acide 4'-[(2-butyl imidazo-(1,2-a)pyridin 3-yl) méthyl] (1,1'-biphényl) 2-carboxylique,
- l'acide 4-[(2-butyl imidazo-(1,2-a)pyridin 3-yl) méthyl] benzoïque,
ainsi que les sels d'addition avec les acides minéraux et organiques ou avec les bases minérales et organiques.

3. Procédé de préparation de produits de formule (I) telle que définie à la revendication 1 dans laquelle R₅ représente un radical 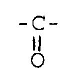 ou méthylène, **caractérisé en ce que** l'on fait réagir un composé de formule (II) : 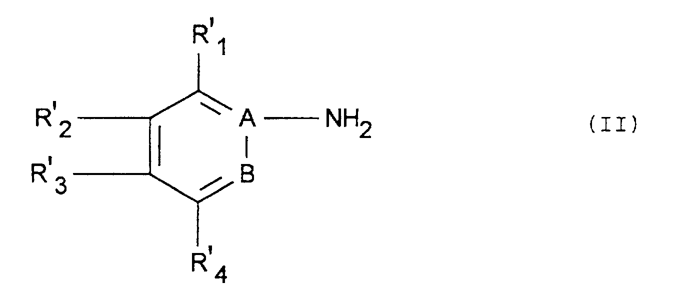 dans laquelle A et B ont la signification indiquée à la revendication 1 et R'₁, R'₂, R'₃ et R'₄ ont les significations indiquées à la revendication 1 respectivement pour R₁, R₂, R₃ et R₄ dans lesquelles les éventuelles fonctions réactives sont éventuellement protégées par des groupements protecteurs, avec un composé de formule (III) : 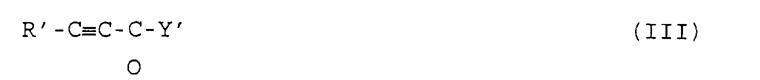 dans laquelle R' et Y' ont les significations indiquées à la revendication 1 pour R et Y dans lesquelles les éventuelles fonctions réactives sont éventuellement protégées par des groupements protecteurs, pour obtenir un produit de formule (IV) 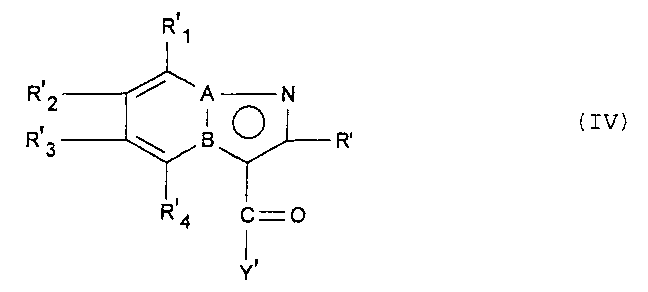 dans laquelle A, B, R', R'₁, R'₂, R'₃, R'₄ et Y' ont les significations indiquées ci-dessus, que l'on soumet éventuellement à une réaction de réduction pour obtenir un produit de formule (V) : 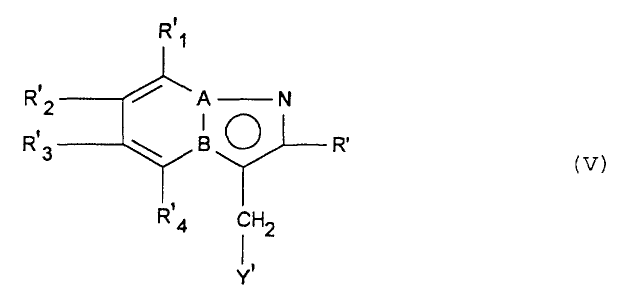 dans laquelle R', R'₁, R'₂, R'₃, R'₄ et Y' ont les significations indiquées ci-dessus, produits de formules (IV) et (V) que l'on traite, si désiré et si nécessaire, à l'une ou plusieurs des réactions suivantes, dans un ordre quelconque :
- une réaction d'élimination des groupements protecteurs que peuvent porter les fonctions réactives protégées,
- une réaction de salification par un acide minéral ou organique ou par une base pour obtenir le sel correspondant,
- une réaction d'hydrogénation du cycle pyridinyl porteur des groupes A et B,
- une réaction de substitution d'un atome d'halogène par un radical cyano,
- une réaction de substitution d'un atome d'halogène par un radical alkyle ou aryle éventuellement substitué,
- une réaction de transformation de fonction cyano en fonction acide,
- une réaction de transformation de fonction cyano en fonction tétrazolyle,
- une réaction d'estérification ou salification de fonction acide,
- une réaction de transformation de radical formyle en radical carbamoyle,
- une réaction de transformation de radical carbamoyle en radical cyano,
- une réaction de saponification de fonction ester en fonction acide,
- une réaction de transformation de fonction alcoxy en fonction hydroxyle,
- une réaction de réduction de la fonction carboxy en fonction alcool,
- une réaction de réduction de la fonction oxo en radical alkylène,
- une réaction d'oxydation de radical alkylène en fonction oxo
- une réaction de transformation de radicaux alkylthio ou arylthio en les radicaux sulfoxyde ou sulfone correspondants,
- une réaction de dédoublement des formes racémiques en produits dédoublés,
lesdits produits de formule (I)ₘ ainsi obtenus, qui correspondent aux produits de formule (I) telle que définie à la revendication 1 dans laquelle R₅ représente un radical 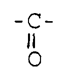 ou méthylène, étant sous toutes les formes isomères possibles racémiques, énantiomères et diastéréoisomères.

4. Procédé de préparation de produits de formule (I) telle que définie à la revendication 1 **caractérisé en ce que** l'on fait réagir un composé de formule (II) : 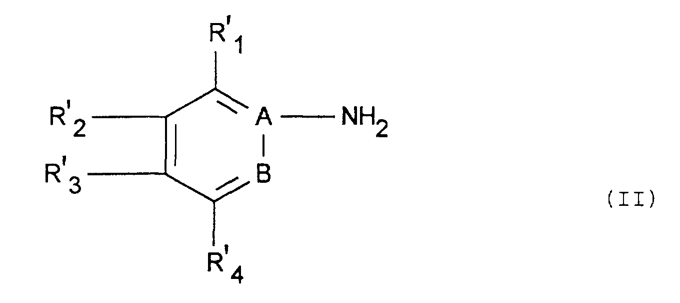 dans laquelle A et B ont la signification indiquée à la revendication 1 et R'₁, R'₂, R'₃ et R'₄ ont les significations indiquées à la revendication 1 respectivement pour R₁, R₂, R₃ et R₄ dans lesquelles les éventuelles fonctions réactives sont éventuellement protégées par des groupements protecteurs, avec un composé de formule (VI) : 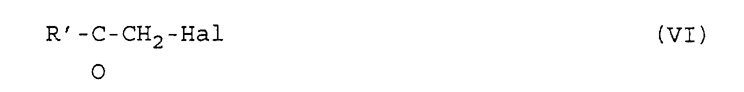 dans laquelle R' a la signification indiquée à la revendication 1 pour R dans laquelle les éventuelles fonctions réactives sont éventuellement protégées par des groupements protecteurs et Hal représente un atome d'halogène, pour obtenir un produit de formule (VII) : 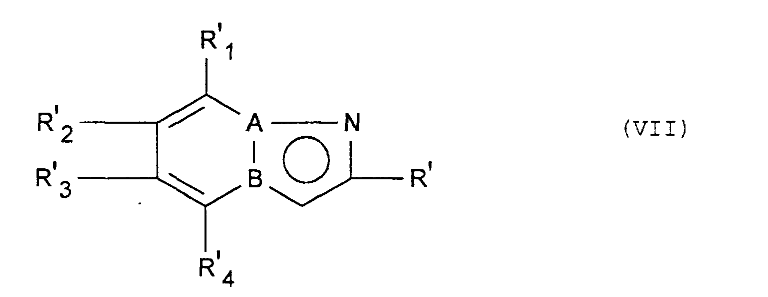 dans laquelle A, B, R', R'₁, R'₂, R'₃ et R'₄ ont les significations indiquées ci-dessus,
que l'on soumet à une réaction d'halogénation pour obtenir un produit de formule (VIII) : 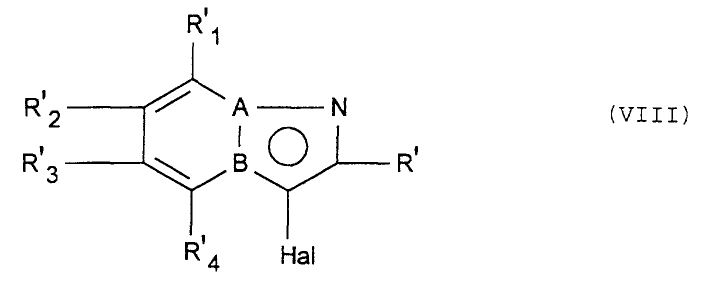 dans laquelle A, B, R', R'₁, R'₂, R'₃ et R'₄ ont les significations indiquées ci-dessus et Hal représente un atome d'halogène, que l'on fait réagir avec le composé de formule (IX) :
Br-Zn-R₅-Y' (IX)
dans laquelle Y' a la signification indiquée à la revendication 1 pour Y dans laquelle les éventuelles fonctions réactives sont éventuellement protégées par des groupements protecteurs et R₅ a la signification indiquée à la revendication 1, pour obtenir un produit de formule (X) : 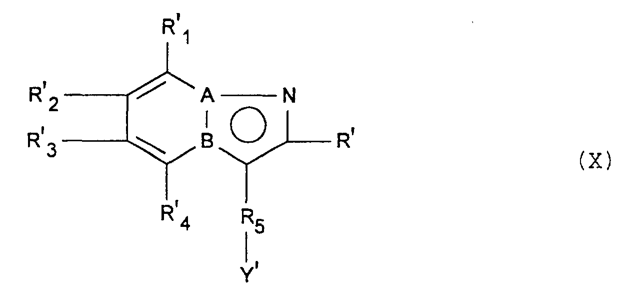 dans laquelle A, B, R', R'₁, R'₂, R'₃, R'₄, R₅ et Y' ont les significations indiquées ci-dessus, produits de formule (X) que l'on traite, si désiré et si nécessaire, à l'une ou plusieurs des réactions suivantes, dans un ordre quelconque :
- une réaction d'élimination des groupements protecteurs que peuvent porter les fonctions réactives protégées,
- une réaction de salification par un acide minéral ou organique ou par une base pour obtenir le sel correspondant,
- une réaction d'hydrogénation du cycle pyridinyle,
- une réaction de substitution d'un atome d'halogène par un radical cyano,
- une réaction de substitution d'un atome d'halogène par un radical aryle éventuellement substitué,
- une réaction de transformation de fonction cyano en fonction acide,
- une réaction de transformation de fonction cyano en fonction tétrazolyle,
- une réaction d'estérification ou salification de fonction acide,
- une réaction de transformation de radical formyle en radical carbamoyle,
- une réaction de transformation de radical carbamoyle en radical cyano,
- une réaction de saponification de fonction ester en fonction acide,
- une réaction de transformation de fonction alcoxy en fonction hydroxyle,
- une réaction de réduction de la fonction carboxy en fonction alcool,
- une réaction de réduction de la fonction oxo en radical alkylène,
- une réaction d'oxydation de radical alkylène en fonction oxo
- une réaction de transformation de radicaux alkylthio ou arylthio en les radicaux sulfoxyde ou sulfone correspondants,
- une réaction de dédoublement des formes racémiques en produits dédoublés,
lesdits produits de formule (I) ainsi obtenus étant sous toutes les formes isomères possibles racémiques, énantiomères et diastéréoisomères.

5. A titre de médicaments, les produits de formule (I) tels que définis aux revendications 1 et 2,
ainsi que les sels d'addition avec les acides minéraux et organiques ou avec les bases minérales et organiques pharmaceutiquement acceptables desdits produits de formule (I).

6. Les compositions pharmaceutiques contenant à titre de principe actif, l'un au moins des médicaments tels que définis à la revendication 5.

7. A titre de produits industriels, les composés de formule (X) telle que définie à la revendication 4 à l'exception des produits dans lesquels R₁, R₂, R₃, R₄ répresentent un atome d'hydrogène
A représente N
R représente -CO
et Y représente le radical phenyle substitué par un atome d'halogène.

8. A titre de produits industriels, les composés de formule (X) telle que définie à la revendication 7 répondant aux formules (IV) et (V) telles que définies à la revendication 3.

## Claims

1. a) Products of formula (I): 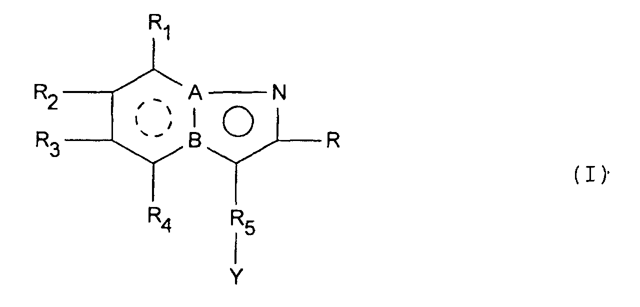 in which:
one of A or B represents a nitrogen atom and the other represents a carbon atom such that the heterobicycle thus formed represents an imidazo pyridine, pyrazolo pyridine or pyrazolo tetrahydro imidazotetrahydropyridine, pyridine radical,
R represents an n-butyl or methyl radical,
R₁, R₂, R₃ and R₄ are such that three of them represent a hydrogen atom and the other represents a hydrogen atom, a carboxy radical which is free or esterified by a linear or branched alkyl radical containing up to 4 carbon atoms,
R₅ represents a methylene radical or a -C=O radical,
and Y represents the phenyl or biphenyl radical substituted by a halogen atom, a cyano, carboxy radical which is free or esterified by an alkyl radical or a tetrazolyl radical, the alkyl radicals being linear or branched and containing up to 4 carbon atoms, with the exception of products in which R₁, R₂, R₃ and R₄ represent a hydrogen atom
A represents N,
R represents an alkyl radical,
R₅ represents -CO
and Y represents the phenyl radical substituted by a halogen atom.
b) The following products:
- (4-bromophenyl) (2-butyl pyrazolo (1,5-a) pyridin-3-yl) methanone
- (4-bromophenyl) (2-butyl 7- methyl pyrazolo (1,5-a) pyridin-3-yl) methanone
said products of formula (I) being in all possible racemic, enantiomeric and diastereoisomeric isomer forms, as well as the addition salts with mineral and organic acids or with mineral and organic bases of said products of formula (I).

2. 2-butyl 3-[(2'-1H-tetrazol 5-yl) (1,1'-biphenyl) 4-yl) methyl] pyrazolo-(1,5-a)pyridine 4-carboxylic acid,
- 4'-[(2-butyl pyrazolo-(1,5-a)pyridin 3-yl) methyl] (1,1'-biphenyl) 2-carboxylic acid,
- 4'-[(2-butyl imidazo-(1,2-a)pyridin 3-yl) methyl] (1,1'-biphenyl) 2-carboxylic acid,
- 4-[(2-butyl imidazo-(1,2-a)pyridin 3-yl) methyl] benzoic acid,
as well as the addition salts with mineral and organic acids or with mineral and organic bases.

3. Process for the preparation of products of formula (I) as defined in claim 1 in which R₅ represents a 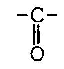 or methylene radical, **characterized in that** a compound of formula (II): 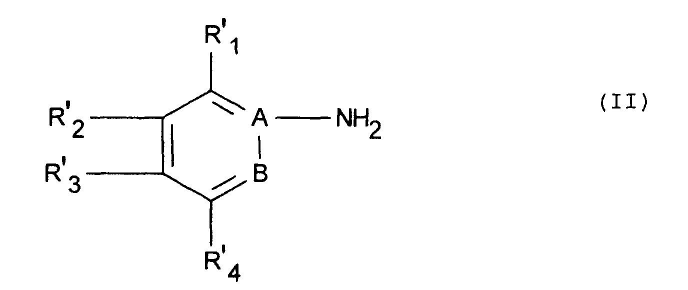 in which A and B have the meaning indicated in claim 1 and R'₁, R'₂, R'₃ and R'₄ have the meanings indicated in claim 1 respectively for R₁, R₂, R₃ and R₄ in which the optional reactive functions are optionally protected by protective groups, is reacted with a compound of formula (III): 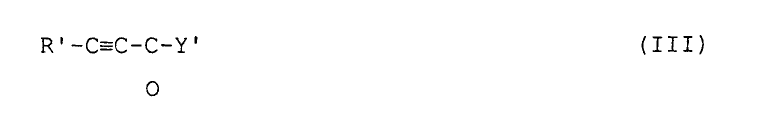 in which R' and Y' have the meanings indicated in claim 1 for R and Y in which the optional reactive functions are optionally protected by protective groups, in order to obtain a product of formula (IV): 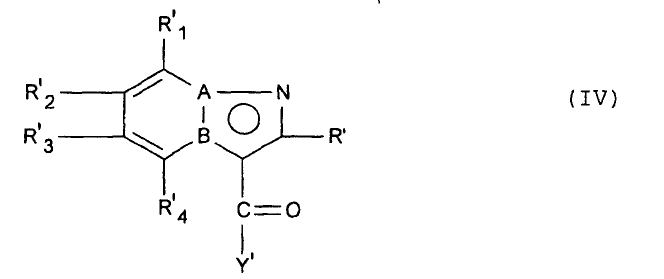 in which A, B, R', R'₁, R'₂, R'₃, R'₄ and Y' have the meanings indicated above, which is optionally subjected to a reduction reaction in order to obtain a product of formula (V): 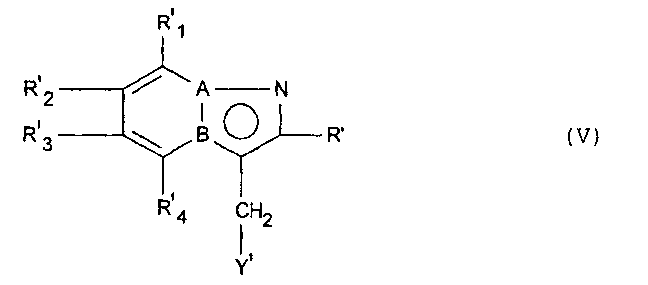 in which R', R'₁, R'₂, R'₃, R'₄ and Y' have the meanings indicated above, which products of formulae (IV) and (V) are treated, if desired and if necessary, by one or more of the following reactions, in any order:
- an elimination reaction of the protective groups which can be carried by the protected reactive functions,
- a salification reaction by a mineral or organic acid or by a base in order to obtain the corresponding salt,
- a hydrogenation reaction of the pyridinyl ring carrying the A and B groups,
- a substitution reaction of a halogen atom by a cyano radical,
- a substitution reaction of a halogen atom by an optionally substituted alkyl or aryl radical,
- a conversion reaction of the cyano function to an acid function,
- a conversion reaction of the cyano function to a tetrazolyl function,
- an esterification or salification reaction of the acid function,
- a conversion reaction of the formyl radical to a carbamoyl radical,
- a conversion reaction of the carbamoyl radical to a cyano radical,
- a saponification reaction of the ester function to an acid function,
- a conversion reaction of the alkoxy function to a hydroxyl function,
- a reduction reaction of the carboxy function to an alcohol function,
- a reduction reaction of the oxo function to an alkylene radical,
- an oxidation reaction of the alkylene radical to an oxo function
- a conversion reaction of the alkylthio or arylthio radicals to corresponding sulphoxide or sulphone radicals,
- a resolution reaction of the racemic forms to resolved products,
said products of formula (I)ₘ thus obtained, which correspond to products of formula (I) as defined in claim 1 in which R₅ represents a 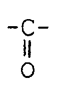 or methylene radical, being in all possible racemic, enantiomeric and diastereoisomeric forms.

4. Process for the preparation of products of formula (I) as defined in claim 1 **characterized in that** a compound of formula (II): 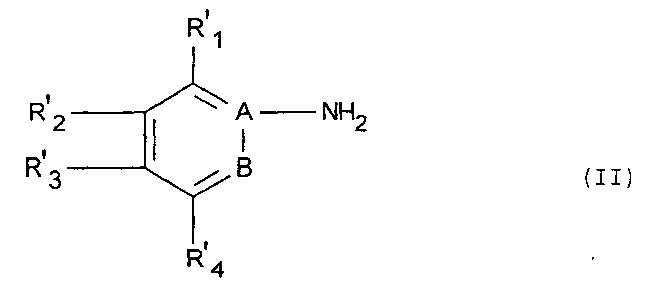 in which A and B have the meaning indicated in claim 1 and R'₁, R'₂, R'₃ and R'₄ have the meanings indicated in claim 1 for R₁, R₂, R₃ and R₄ respectively in which the optional reactive functions are optionally protected by protective groups, is reacted with a compound of formula (VI) : 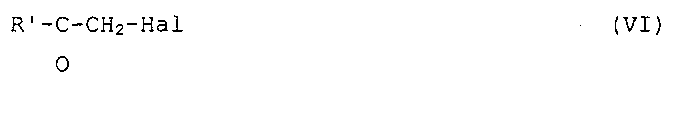 in which R' has the meaning indicated in claim 1 for R in which the optional reactive functions are optionally protected by protective groups and Hal represents a halogen atom, in order to obtain a product of formula (VII): 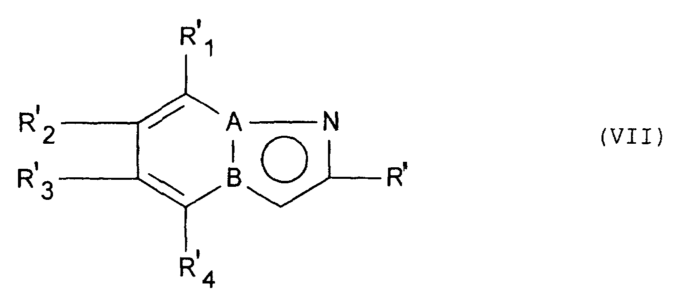 in which A, B, R', R'₁, R'₂, R'₃ and R'₄ have the meanings indicated above,
which is subjected to a halogenation reaction in order to obtain a product of formula (VIII): 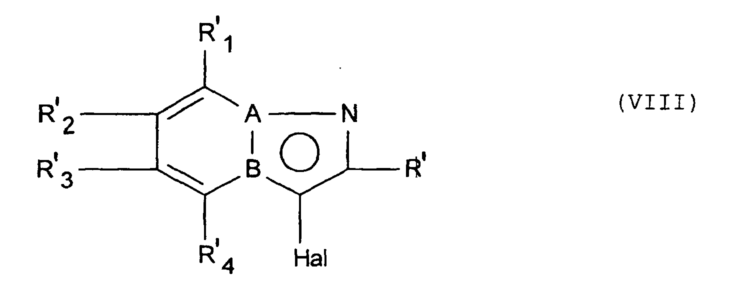 in which A, B, R', R'₁, R'₂, R'₃ and R'₄ have the meanings indicated above and Hal represents a halogen atom, which is reacted with the compound of formula (IX):
Br-Zn-R₅-Y' (IX)
in which Y' has the meaning indicated in claim 1 for Y in which the optional reactive functions are optionally protected by protective groups and R₅ has the meaning indicated in claim 1, in order to obtain a product of formula (X): 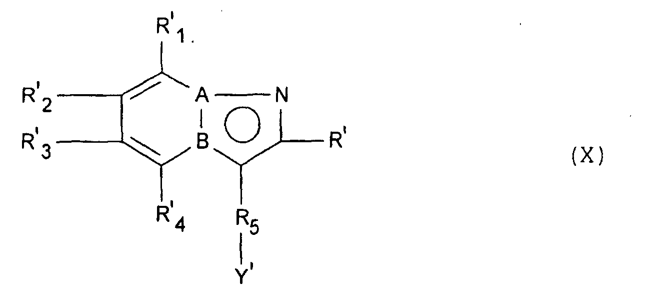 in which A, B, R', R'₁, R'₂, R'₃, R'₄, R₅ and Y' have the meanings indicated above, which products of formula (X) are treated, if desired and if necessary, by one or more of the following reactions, in any order:
- an elimination reaction of the protective groups which can be carried by the protected reactive functions,
- a salification reaction by a mineral or organic acid or by a base in order to obtain the corresponding salt,
- a hydrogenation reaction of the pyridinyl ring,
- a substitution reaction of a halogen atom by a cyano radical,
- a substitution reaction of a halogen atom by an optionally substituted aryl radical,
- a conversion reaction of the cyano function to an acid function,
- a conversion reaction of the cyano function to a tetrazolyl function,
- an esterification or salification reaction of the acid function,
- a conversion reaction of the formyl radical to a carbamoyl radical,
- a conversion reaction of the carbamoyl radical to a cyano radical,
- a saponification reaction of the ester function to an acid function,
- a conversion reaction of the alkoxy function to a hydroxyl function,
- a reduction reaction of the carboxy function to an alcohol function,
- a reduction reaction of the oxo function to an alkylene radical,
- an oxidation reaction of the alkylene radical to an oxo function
- a conversion reaction of the alkylthio or arylthio radicals to corresponding sulphoxide or sulphone radicals,
- a resolution reaction of the racemic forms to resolved products, said products of formula (I) thus obtained being in all possible racemic, enantiomeric and diastereoisomeric isomer forms.

5. As medicaments, the products of formula (I) as defined in claims 1 and 2, as well as the addition salts with pharmaceutically acceptable mineral and organic acids or mineral and organic bases of said products of formula (I).

6. The pharmaceutical compositions containing as active ingredient, at least one of the medicaments as defined in claim 5.

7. As industrial products, the compounds of formula (X) as defined in claim 4 with the exception of products in which R₁, R₂, R₃ and R₄ represent a hydrogen atom
A represents N,
R represents -CO and Y represents the phenyl radical substituted by a halogen atom.

8. As industrial products, the compounds of formula (X) as defined in claim 7 corresponding to formulae (IV) and (V) as defined in claim 3.

## Patentansprüche

1. a) Produkte der Formel (I) 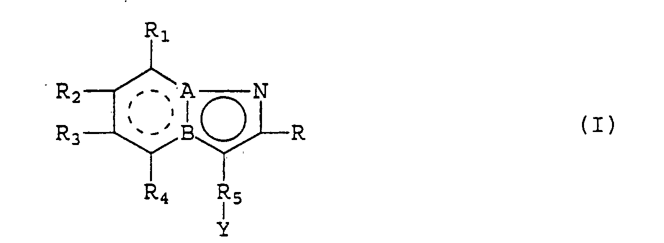 in der
eines von A oder B ein Stickstoffatom und das andere ein Kohlenstoffatom in der Weise darstellt, daß der auf diese Weise gebildete Heterocyclus einen Rest Imidazo-pyridin, Pyrazolo-pyridin oder Pyrazolo-tetrahydro-imidazotetrahydropyridin, Pyridin bedeutet,
R ein Rest n-Butyl oder Methyl ist,
R₁, R₂, R₃ und R₄ so beschaffen sind, daß drei von ihnen ein Wasserstoffatom darstellen und das andere ein Wasserstoffatom, einen Rest Carboxy, frei oder verestert durch einen geraden oder verzweigten Rest Alkyl mit höchstens 4 Kohlenstoffatomen, bedeutet, R₅ ein Rest Methylen oder ein Rest -C=O ist, und
Y einen Rest Phenyl oder Biphenyl darstellt, substituiert durch ein Halogenatom, einen Rest Cyano, Carboxy, frei oder verestert durch einen Rest Alkyl, oder einen Rest Tetrazolyl, wobei die Reste Alkyl gerade oder verzweigt sind und höchstens 4 Kohlenstoffatome umfassen, mit Ausnahme der Produkte, in denen R₁, R₂, R₃ und
R₄ ein Wasserstoffatom darstellen,
A N darstellt,
R einen Rest Alkyl darstellt,
R₅ -CO darstellt und
Y den Rest Phenyl darstellt, substituiert durch ein Halogenatom; b) die. folgenden Produkte:
- (4-Bromphenyl)-[2-butyl-pyrazolo(1,5-a)-pyridin-3-yl]-methanon,
- (4-Bromphenyl)-[2-butyl-7-methyl-pyrazolo(1,5-a)-pyridin-3-yl]-methanon,
wobei die genannten Produkte der Formel (I) in allen möglichen isomeren racemischen Formen, Enantiomeren und Diastereoisomeren vorliegen können, sowie die Additionssalze der genannten Produkte der Formel (I) mit Mineralsäuren und organischen Säuren oder mit Mineralbasen und organischen Basen.

2. 2-Butyl-3-{[(2'-1H-tetrazol-5-yl)-(1,1'-biphenyl)-4-yl]-methyl)-pyrazolo(1,5-a)-pyridin-4-carbonsäure,
- 4'-{[2-Butyl-pyrazolo(1,5-a)-pyridin-3-yl]-methyl)-(1,1'-biphenyl)-2-carbonsäure,
- 4'-{[2-Butyl-imidazo(1,2-a)-pyridin-3-yl]-methyl}-(1,1'-biphenyl)-2-carbonsäure,
- 4-{[2-Butyl-imidazo(1,2-a)-pyridin-3-yl]-methyl}-benzoesäure, sowie die Additionssalze mit Mineralsäuren und organischen Säuren oder mit Mineralbasen und organischen Basen.

3. Verfahren zur Herstellung der Produkte der Formel (I) wie in Anspruch 1 definiert, worin R₅ einen Rest 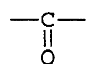 oder Methylen darstellt, **dadurch gekennzeichnet, daß** man eine Verbindung der Formel (II) 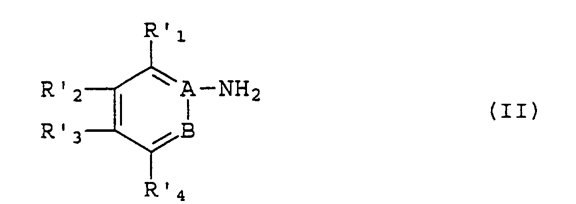 in der A und B die in Anspruch 1 angegebenen Bedeutungen besitzen und R'₁, R'₂, R'₃ und R'₄ die jeweils für R₁, R₂, R₃ und R₄ genannten Bedeutungen besitzen, in denen die eventuellen reaktiven Funktionen gegebenenfalls durch Schutzgruppen geschützt sind, mit einer Verbindung der Formel (III) 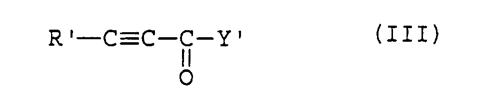 zur Reaktion bringt, in der R' und Y' die in Anspruch 1 für R und Y angegebenen Bedeutungen besitzen, in denen die eventuellen reaktiven Funktionen gegebenenfalls durch Schutzgruppen geschützt sind, um ein Produkt der Formel (IV) 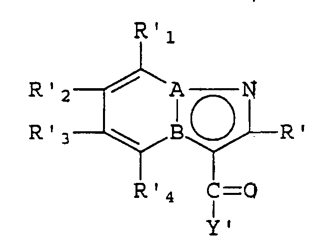 zu erhalten, in der A, B, R', R'₁, R'₂, R'₃, R'₄ und Y' die oben angegebenen Bedeutungen besitzen, das man gegebenenfalls einer Reduktionsreaktion unterzieht, um ein Produkt der Formel (V) 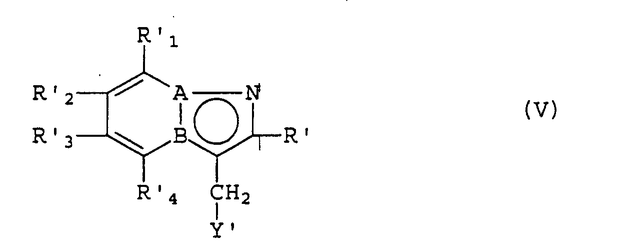 zu erhalten, in der R', R'₁, R'₂, R'₃, R'₄ und Y' die oben angegebenen Bedeutungen besitzen, wobei man die Produkte der Formeln (IV) oder (V), wenn gewünscht und wenn erforderlich, mit einer oder mehreren der nachfolgenden Reaktionen in irgendeiner Reihenfolge behandelt:
- einer Reaktion zur Entfernung der Schutzgruppen, die die geschützten reaktiven Funktionen tragen können,
- einer Reaktion zur Salzbildung durch eine Mineralsäure oder organische Säure oder durch eine Base, um das entsprechende Salz zu erhalten,
- einer Reaktion zur Hydrierung des Pyridinyl-Ringes, der die Gruppen A und B trägt,
- einer Reaktion zur Substitution eines Halogenatoms durch einen Rest Cyano,
- einer Reaktion zur Substitution eines Halogenatoms durch einen gegebenenfalls substituierten Rest Alkyl oder Aryl,
- einer Reaktion zur Umwandlung der Cyanofunktion in die Säurefunktion,
- einer Reaktion zur Umwandlung der Cyanofunktion in die Tetrazolyl-Funktion,
- einer Reaktion zur Veresterung oder Salzbildung der Säurefunktion,
- einer Reaktion zur Umwandlung des Restes Formyl in den Rest Carbamoyl,
- einer Reaktion zur Umwandlung des Restes Carbamoyl in den Rest Cyano,
- einer Reaktion zur Verseifung der Esterfunktion in die Säurefunktion,
- einer Reaktion zur Umwandlung der Alkoxyfunktion in die Hydroxylfunktion,
- einer Reaktion zur Reduktion der Carboxyfunktion in die Alkoholfunktion,
- einer Reaktion zur Reduktion der Oxo-Funktion in den Rest Alkylen,
- einer Reaktion zur Oxidation des Restes Alkylen in die Oxo-Funktion,
- einer Reaktion zur Umwandlung der Reste Alkylthio oder Arylthio in die entsprechenden Reste Sulfoxid oder Sulfon,
- einer Reaktion zur Aufspaltung der racemischen Formen in die Spaltprodukte,
wobei die auf diese Weise erhaltenen Produkte der Formel (I)ₘ, die den wie in Anspruch 1 definierten Produkten der Formel (I) entsprechen, in denen R₅ einen Rest 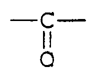 oder Methylen darstellt, in allen möglichen isomeren racemischen Formen, Enantiomeren und Diastereoisomeren vorliegen können.

4. Verfahren zur Herstellung der Produkte der Formel (I) wie in Anspruch 1 definiert, **dadurch gekennzeichnet, daß** man eine Verbindung der Formel (II) 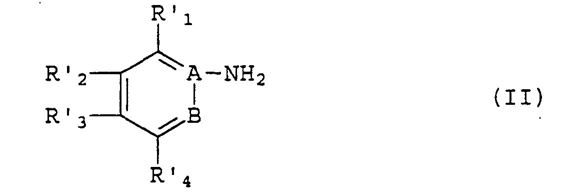 in der A und B die in Anspruch 1 angegebenen Bedeutungen besitzen und R'₁, R'₂, R'₃ und R'₄ die jeweils für R₁, R₂, R₃ und R₄ genannten Bedeutungen besitzen, in denen die eventuellen reaktiven Funktionen gegebenenfalls durch Schutzgruppen geschützt sind, mit einer Verbindung der Formel (VI) 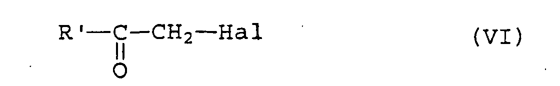 zur Reaktion bringt, in der R' die in Anspruch 1 für R angegebene Bedeutung besitzt, in denen die eventuellen reaktiven Funktionen gegebenenfalls durch Schutzgruppen geschützt sind und Hal ein Halogenatom ist, um ein Produkt der Formel (VII) 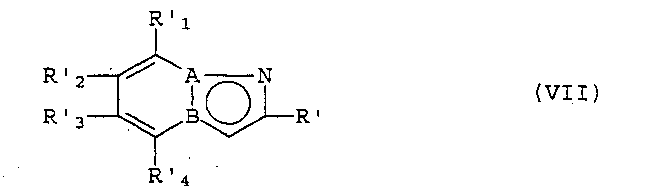 zu erhalten, in der A, B, R', R'₁, R'₂, R'₃ und R'₄ die oben angegebenen Bedeutungen besitzen, das man einer Reaktion zur Halogenierung unterzieht, um ein Produkt der Formel (VIII) 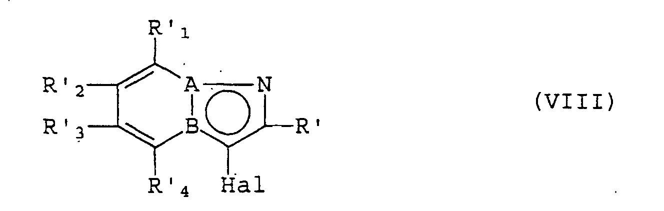 zu erhalten, in der A, B, R', R'₁, R'₂, R'₃ und R'₄ die oben angegebenen Bedeutungen besitzen und Hal ein Halogenatom ist, das man mit der Verbindung der Formel (IX)
Br-Zn-R₅-Y' (IX)
zur Reaktion bringt, in der Y' die in Anspruch 1 für Y angegebene Bedeutung besitzt, worin die eventuellen reaktiven Funktionen gegebenenfalls durch Schutzgruppen geschützt sind und R₅ die in Anspruch 1 angegebene Bedeutung besitzt, um ein Produkt der Formel (X) 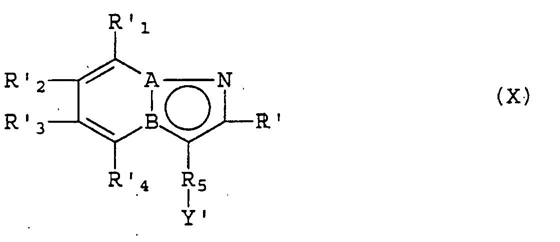 zu erhalten, in der
A, B, R', R'₁, R'₂, R'₃, R'₄ and Y' die oben angegebenen Bedeutungen besitzen, wobei man die Produkte der Formel (X), wenn gewünscht und wenn erforderlich, mit einer oder mehreren der nachfolgenden Reaktionen in irgendeiner Reihenfolge behandelt:
- einer Reaktion zur Entfernung der Schutzgruppen, die die geschützten reaktiven Funktionen tragen können,
- einer Reaktion zur Salzbildung durch eine Mineralsäure oder organische Säure oder durch eine Base, um das entsprechende Salz zu erhalten,
- einer Reaktion zur Hydrierung des Pyridinyl-Ringes,
- einer Reaktion zur Substitution eines Halogenatoms durch einen Rest Cyano,
- einer Reaktion zur Substitution eines Halogenatoms durch einen gegebenenfalls substituierten Rest Aryl,
- einer Reaktion zur Umwandlung der Cyanofunktion in die Säurefunktion,
- einer Reaktion zur Umwandlung der Cyanofunktion in die Tetrazolyl-Funktion,
- einer Reaktion zur Veresterung oder Salzbildung der Säurefunktion,
- einer Reaktion zur Umwandlung des Restes Formyl in den Rest Carbamoyl,
- einer Reaktion zur Umwandlung des Restes Carbamoyl in den Rest Cyano,
- einer Reaktion zur Verseifung der Esterfunktion in die Säurefunktion,
- einer Reaktion zur Umwandlung der Alkoxyfunktion in die Hydroxylfünktion,
- einer Reaktion zur Reduktion der Carboxyfunktion in die Alkoholfunktion,
- einer Reaktion zur Reduktion der Oxo-Funktion in den Rest Alkylen,
- einer Reaktion zur Oxidation des Restes Alkylen in die Oxo-Funktion,
- einer Reaktion zur Umwandlung der Reste Alkylthio oder Arylthio in die entsprechenden Reste Sulfoxid oder Sulfon,
- einer Reaktion zur Aufspaltung der racemischen Formen in die Spaltprodukte,
wobei die auf diese Weise erhaltenen Produkte der Formel (I) in allen möglichen isomeren racemischen Formen, Enantiomeren und Diastereoisomeren vorliegen können.

5. Als Arzneimittel die Produkte der Formel (I) wie in den Ansprüchen 1 und 2 definiert, sowie die Additionssalze der genannten Produkte der Formel (I) mit pharmazeutisch akzeptablen Mineralsäuren und organischen Säuren oder mit Mineralbasen und organischen Basen.

6. Pharmazeutische Zusammenset zungen, enthaltend als Wirkstoff mindestens eines der Arzneimittel wie in Anspruch 5 definiert.

7. Als industrielle Produkte die Verbindungen der Formel (X) wie in Anspruch 4 definiert, mit Ausnahme der Produkte, in denen R₁, R₂, R₃ und R₄ ein Wasserstoffatom darstellen,
A N darstellt,
R -CO darstellt und
Y den Rest Phenyl darstellt, substituiert durch ein Halogenatom.

8. Als industrielle Produkte die Verbindungen der Formel (X) wie in Anspruch 7 definiert, die den Formeln (IV) und (V) entsprechen, wie in Anspruch 3 definiert.
